# EUROPEAN PATENT APPLICATION

(11) **EP 4 343 005 A1**
(43) Date of publication of application: **27.03.2024**
(21) Application number: 23816241.6
(22) Date of filing: 08.05.2023
(51) Int. Cl.: C12Q 1/689, C12Q 1/686

(54) **PRIMER SET FOR IDENTIFYING BACTERIAL SPECIES IN PROBIOTICS COMPOSITION AND METHOD FOR IDENTIFYING BACTERIAL SPECIES USING SAME**

(30) Priority: 31.05.2022 KR 20220067077
(71) Applicant: Chongkundang Healthcare Corp., Dangjin-si, Chungcheongnam-do 31816 (KR)
(72) Inventor: KIM, Myung-Soo, Namyangju-si, Gyeonggi-do 12151 (KR); KANG, Eun Young, Seoul 04746 (KR); LIM, Jong-Hyun, Gimpo-si, Gyeonggi-do 10099 (KR); KIM, Byung-Yong, Anyang-si, Gyeonggi-do 14017 (KR)
(74) Representative: Vossius & Partner Patentanwälte Rechtsanwälte mbB
(86) International application number: PCT/KR2023/006187
(87) International publication number: WO 2023/234577

(57) **Abstract**

The present invention relates to a primer set for discrimination of bacterial species in a probiotic composition and a method for discriminating bacterial species using the same. Specifically, in the present invention, a primer set targeting the ITS (internal transcribed spacer) region between the 16S rRNA and 23S rRNA of probiotic species and pathogenic bacterial species has been constructed, it has been confirmed that 21 probiotic species and 4 pathogenic bacterial species can be discriminated with high resolution and accuracy by performing metagenomic analysis of a probiotic composition using the same, and the primer set can be usefully used to discriminate bacterial species in a probiotic composition.

## Description

### [Technical Field]

The present invention relates to a primer set for discrimination of bacterial species in a probiotic composition and a method for discriminating bacterial species using the same.

### [Background Art]

Probiotics are known to have beneficial health effects on the host when consumed in appropriate amounts. Scientific evidence for the beneficial effects of probiotics on human health is continuously accumulated in various areas such as alleviation of immune disorders, inflammatory bowel disease, type 2 diabetes, and arteriosclerosis.

However, there is a lack of objective quality information on probiotic-related products, and there have been cases in Korea where it has been found that the strains actually used in products are different from the strains reported. In a case where a strain other than the initially approved strain is used in a product, the safety and functionality of the strain cannot be guaranteed, so accurate discrimination of the probiotic strain used in the product is necessary, and it is important to secure safety based on this.

In Korea, probiotics are classified as health functional food and are managed under the Health Functional Foods Act. Raw materials having functionality are classified into raw materials (notified raw materials) notified by the Minister of the Ministry of Food and Drug Safety (hereinafter referred to as Minister of Food and Drug Safety) or separately approved raw materials (individually approved raw materials). Currently, there are a total of 19 notified strains that can be used as probiotics in Korea.

Since the efficacy of probiotics is strain specific, the FAO and WHO probiotic product guidelines published in 2002 states that accurate identification of a strain is greatly important for correlation with the strain's efficacy and for accurate tracking and epidemiological studies. This is because even strains of the same genus and species have remarkably great differences in the efficacy of probiotics, particularly the neurological effects, immunomodulatory effects, endocrinological effects, production of specific bioactive substances, and the like. Therefore, technology to identify specific strains within probiotic products is essential not only for product management but also for tracking the strains in the human body after ingestion of products containing the strains. Recently, as it has become easier to analyze the whole genome of selected strains, quantitative and qualitative analysis of specific strains has become possible through the design of strain-specific primers using comparative genome technology and PCR (polymerase chain reaction) using the same. However, primers for each bacterial species are required, and the experiment process is cumbersome when the analysis is performed on multiple bacterial species.

The Korea Food and Drug Safety Evaluation Institute has distributed a safety evaluation guide for probiotics, functional raw materials for health functional foods, in 2021 and presented a safety evaluation method through NGS-based metagenomic/metashotgun technology to identify the type of strain within a probiotic product or complex strain and analyze its composition. Therefore, microbial species in products are identified using the 16S metagenomic NGS analysis method provided by the NGS analysis service provider. Among them, MTP (microbiome taxonomic profiling), a representative service, targets 16S V3V4, which is about 460 bp in length. However, to secure a long sequence, 250×2 paired read analysis is required to be performed using expensive equipment such as MiSeq or HiSeq, which requires an analysis period of about two weeks and a cost of KRW 200,000 or more per product. PCC (probiotics contents certificate), another analysis service, performs bacterial species identification through the WGS metagenomic analysis method, which requires expensive equipment such as HiSeq to acquire sufficient coverage for analysis, and requires an analysis period of about one month and a cost of about KRW 1 million or more per product.

In addition, MTP, which is based on the 16S metagenomic analysis method, cannot distinguish between similar bacterial species having a 16S rRNA sequence similarity of 98% or more, such as *Lactobacillus helveticus* and *Lactobacillus crispatus,* and is thus limited to genus-level analysis. PCC, a WGS metagenomic analysis method, allows species-level analysis, but the detection limit is about 1%, so the detection power for bacterial species contained at low ratios is low.

Accordingly, the present inventors have strived to develop a probiotic product quality control technology that does not require expensive analysis equipment, is not cumbersome in analysis techniques, and can secure economic feasibility such as shortening of analysis time and cost reduction, and as a result, constructed a primer set targeting the ITS (internal transcribed spacer) region having a length of about 200 bp between the 16S rRNA and 23S rRNA of probiotic species and pathogenic bacterial species, and confirmed that 21 probiotic species and 4 pathogenic bacterial species can be discriminated with high resolution and accuracy by performing metagenomic analysis of probiotic compositions using the same. Therefore, by revealing that the primer and the method for discriminating bacterial species using the same can be usefully used to discriminate the bacterial species in probiotic products and objective and reasonable information on the quality information and safety of probiotic products can be provided through this, the present application has been concluded.

### [Citation List]

### [Patent Literature]

[Patent Literature 1]
   Korean Patent Publication No. 10-2020-0029689
[Patent Literature 2]
   Korean Patent Publication No. 10-2020-0027900

### [Non Patent Literature]

[Non Patent Literature 1]
   Seong Young-je, Park Myung-soo. Current status of domestic and international probiotic product development. Food Science and Industry (Vol.52 No.3), 229-240
[Non Patent Literature 2]
   Lee Joo-hoon. Study on probiotic genetic characteristics and safety confirmation method. Ministry of Food and Drug Safety (2010-11), TRKO202100007735

### [Summary of Invention]

### [Technical Problem]

Existing methods for discriminating probiotic species in probiotic products have the problems of requiring a long analysis period and high cost and making species-level analysis difficult.

Accordingly, an object of the present invention is to solve the problems and provide a primer set that can simultaneously discriminate various bacterial species, such as probiotics and pathogenic bacteria, in probiotic products, and a method for discriminating bacterial species in probiotic products using the same.

### [Solution to Problem]

In order to achieve the object, the present invention provides a primer set for discrimination of bacterial species in a probiotic composition, which includes primers represented by SEQ ID NOS: 1 to 9.

The present invention also provides a composition for discrimination of bacterial species in a probiotic composition, which includes the primer set.

In addition, the present invention provides a method for discriminating bacterial species in a probiotic composition, which includes:
1) isolating DNA from the probiotic composition;
2) performing PCR using the DNA isolated in step 1) as a template and the primer set to amplify a target sequence; and
3) detecting the amplification product obtained in step 2).

### [Advantageous Effects of Invention]

In the present invention, it has been confirmed that by constructing a primer set targeting the ITS (internal transcribed spacer) region having a length of about 200 bp between the 16S rRNA and 23S rRNA of probiotic species and pathogenic bacterial species and performing metagenomic analysis of a probiotic composition using the same, 21 probiotic species and 4 pathogenic bacterial species can be discriminated with high resolution and accuracy, and that the primer set can be usefully used to discriminate bacterial species in a probiotic composition.

In particular, the primer set according to the present invention targets the ITS region having a length of about 200 bp, so 300×1 single read data is sufficient, sufficient data can be acquired using low-cost NGS equipment including iSeq100, probiotic products can be analyzed in about 3 days and at a cost of about KRW 55,000 per product through this, and the primer set has an effect of increasing economic feasibility compared to existing technologies.

The primer set targets a common ITS region to simultaneously discriminate various bacterial species contained in probiotic products at the species level, so it has an effect of eliminating the inconvenience of constructing a primer for each bacterial species.

Therefore, the primer set and the method for discriminating bacterial species in a probiotic composition using the same according to the present invention can be used to provide objective and reasonable information on the quality information and safety of probiotic products.

### [Description of Embodiments]

Hereinafter, the present invention will be described in more detail.

The present invention provides a primer set for discrimination of bacterial species in a probiotic composition, which includes primers represented by SEQ ID NOS: 1 to 9.

The present invention also provides a composition for discrimination of bacterial species in a probiotic composition, which includes the primer set.

As used herein, the term "probiotics" refers to live microorganisms, for example, bacteria, that confer health benefits. The term "probiotic composition" refers to a composition containing probiotics and may be used interchangeably with "probiotic product."

As used herein, the term "polymerase chain reaction (PCR)" is a commonly used method of exponentially amplifying a starting nucleic acid material by sequentially synthesizing nucleic acids using polymerase.

As used herein, the term "primer" refers to a single-stranded oligonucleotide sequence complementary to the strand of nucleic acid to be replicated, serves as a starting point for the synthesis of primer extension products that are replicated and amplified by PCR, and is divided into forward primers and reverse primers.

In the present invention, the primer set includes a forward primer and a reverse primer, specifically a forward primer represented by SEQ ID NO: 1 and reverse primers represented by SEQ ID NOS: 2 to 9.

The sequence of the primer does not have to be completely complementary to a partial sequence of the template, and is only required to be sufficiently complementary within the range in which the primer can hybridize with the template and exhibits its original function. Therefore, the primer set according to the present invention may be a perfectly complementary sequence to the nucleotide sequence of the template, or may not be a perfectly complementary sequence to the nucleotide sequence of the template but may be a substantially complementary sequence within the range in which the sequence does not interfere with the amplification of the target gene for which the present disclosure aims.

"Substantially complementary" herein means that two nucleic acid strands that are sufficiently complementary in sequence are annealed and form a stable duplex. The complementarity does not have to be perfect. For example, there may be several base pair mismatches between two nucleic acids. However, in a case where the number of mismatches is so great that hybridization does not occur even under minimally stringent hybridization conditions, the sequences are not substantially complementary sequences. When two sequences are interpreted as "substantially complementary" in the present invention, it means that the sequences are sufficiently complementary so as to hybridize with each other under selected reaction conditions, such as stringent hybridization conditions. The relation between sufficient complementarity of nucleic acids to achieve specificity and stringency of hybridization is well known in the art. Two substantially complementary strands may be, for example, completely complementary or may contain from one to a number of mismatches, for example, as long as they sufficiently allow for differences between the paired and unpaired sequences. Accordingly, a "substantially complementary" sequence may mean a sequence that has a base pair complementarity of 100%, 95%, 90%, 80%, 75%, 70%, 60%, 50% or more, or any percentage between the numbers in the double-stranded region.

The primer may further include an adapter sequence at the 5' end, specifically may be a primer represented by SEQ ID NOS: 10 to 18. More specifically, the primer may be a primer represented by SEQ ID NO: 10, which further includes an adapter sequence at the 5' end of the primer represented by SEQ ID NO: 1, a primer represented by SEQ ID NO: 11, which further includes an adapter sequence at the 5' end of the primer represented by SEQ ID NO: 2, a primer represented by SEQ ID NO: 12, which further includes an adapter sequence at the 5' end of the primer represented by SEQ ID NO: 3, a primer represented by SEQ ID NO: 13, which further includes an adapter sequence at the 5' end of the primer represented by SEQ ID NO: 4, a primer represented by SEQ ID NO: 14, which further includes an adapter sequence at the 5' end of the primer represented by SEQ ID NO: 5, a primer represented by SEQ ID NO: 15, which further includes an adapter sequence at the 5' end of the primer represented by SEQ ID NO: 6, a primer represented by SEQ ID NO: 16, which further includes an adapter sequence at the 5' end of the primer represented by SEQ ID NO: 7, a primer represented by SEQ ID NO: 17, which further includes an adapter sequence at the 5' end of the primer represented by SEQ ID NO: 8, and a primer represented by SEQ ID NO: 18, which further includes an adapter sequence at the 5' end of the primer represented by SEQ ID NO: 8.

In the present invention, the bacterial species may be probiotic species and pathogenic bacterial species. Specifically, the probiotic species may be bacterial species approved and notified as a health functional food by the Ministry of Food and Drug Safety and bacterial species that can be used in food.

More specifically, the bacterial species approved and notified as a health functional food by the Ministry of Food and Drug Safety may be *Lactobacillus acidophilus, Lactobacillus gasseri, Lactobacillus delbruecki* ssp. *Bulgaricus, Lactobacillus helveticus, Lacticaseibacillus casei, Lacticaseibacillus paracasei, Lacticaseibacillus rhamnosus, Limosilactobacillus fermentum, Limosilactobacillus reuteri, Lactiplantibacillus plantarum, Ligilactobacillus salivarius, Lactococcus lactis, Enterococcus faecium, Enterococcus faecalis, Streptococcus thermophilus, Bifidobacterium bifidum, Bifidobacterium breve, Bifidobacterium longum,* and *Bifidobacterium animalis* ssp. *lactis.*

The bacterial species that can be used in food may be *Latilactobacillus curvatus* and *Bacillus coagulans.*

The pathogenic bacterial species may be *Escherichia coli, Salmonella spp., Staphylococcus aureus,* and *Pseudomonas aeruginosa.*

In the present invention, the primer set targets the ITS (internal transcribed spacer) region of the bacterial species, specifically the ITS region having a length of about 200 bp. Accordingly, the bacterial species contained in a probiotic product can be simultaneously discriminated. As the ITS region having a length of about 200 bp is targeted, analysis is possible using low-cost NGS equipment such as iSeq100, and the cost and period required to discriminate bacterial species in a probiotic product can be reduced.

In the present invention, the composition may contain reagents for PCR.

The reagents for PCR may include any reagents widely known in the art. For example, the reagents may include a heat-resistant DNA polymerase, dNTPs, and a buffer. The dNTPs include dATP, dCTP, dGTP, and dTTP, and a commercially available heat-resistant polymerase such as Taq DNA polymerase can be used as the heat-resistant DNA polymerase.

In addition, the present invention provides a method for discriminating bacterial species in a probiotic composition, which includes:
1) isolating DNA from the probiotic composition;
2) performing PCR using the DNA isolated in step 1) as a template and the primer set according to the present invention to amplify a target sequence; and
3) detecting the amplification product obtained in step 2).

In the method of the present invention, the primer, bacterial species and the like are the same as those described above, so the above contents are used for specific description.

In the method of the present invention, DNA isolation in step 1) may be performed according to a method commonly used in the art, and may be performed using a commercially available DNA extraction kit.

In the method of the present invention, PCR in step 2) may be performed according to a method commonly used in the art, and may be performed using commercially available reagents for PCR.

The method of the present invention may further include performing index PCR using the amplification product obtained in step 2) as a template. By performing the index PCR, an index can be attached to the amplification product.

In the method of the present invention, detection of the amplification product in step 3) may be performed through sequencing, for example, next generation sequencing (NGS). In particular, sequencing can be performed using small NGS equipment such as iSeq100, and sequencing cost can be reduced and the speed can be improved.

### [Examples]

Hereinafter, the present invention will be described in detail with reference to Examples.

However, the following Examples only illustrate the present invention, and the contents of the present invention are not limited to the following Examples.

### <Example 1> Construction of primer set for discrimination of bacterial species in probiotic product

Constructed were primers that could discriminate probiotic species used in probiotic products, which were 19 probiotic species notified by the Ministry of Food and Drug Safety and 2 probiotic species added in food, and 4 pathogenic bacterial species.

Specifically, complete genome information of 19 probiotic species notified by the Ministry of Food and Drug Safety, 2 probiotic species added in food, and 4 pathogenic bacterial species was acquired from the GenBank database of the U.S. National Center for Biological Information (NCBI), as shown in [Table 1] below. Accession numbers in the NCBI GeneBank database for the bacterial species are as shown in [Table 2] below.

**[Table 1]**

| No. | Division | Species name | Strain genome number |
|---|---|---|---|
| 1 | Species notified by Ministry of Food and Drug Safety | *Lactobacillus acidophilus* | 9 |
| 2 | | *Lactobacillus gasseri* | 11 |
| 3 | | *Lactobacillus helveticus* | 20 |
| 4 | | *Lacticaseibacillus casei* | 5 |
| 5 | | *Lacticaseibacillus paracasei* | 49 |
| 6 | | *Lacticaseibacillus rhamnosus* | 34 |
| 7 | | *Limosilactobacillus fermentum* | 30 |
| 8 | | *Limosilactobacillus reuteri* | 26 |
| 9 | | *Lactiplantibacillus plantarum* | 152 |
| 10 | | *Ligilactobacillus salivarius* | 12 |
| 11 | | *Lactococcus lactis* | 41 |
| 12 | | *Enterococcus faecium* | 226 |
| 13 | | *Enterococcus faecalis* | 75 |
| 14 | | *Streptococcus thermophilus* | 73 |
| 15 | | *Bifidobacterium bifidum* | 9 |
| 16 | | *Bifidobacterium breve* | 43 |
| 17 | | *Bifidobacterium animalis ssp. Lactis* | 11 |
| 18 | | *Lactobacillus delbruecki ssp. Bulgaricus* | 7 |
| 19 | | *Bifidobacterium longum* | 51 |
| 20 | Species added in food | *Latilactobacillus curvatus* | 12 |
| 21 | | *Bacillus coagulans* | 14 |
| 22 | Pathogenic bacterial species | *Escherichia coli* | 1901 |
| 23 | | *Salmonella spp.* | 1067 |
| 24 | | *Staphylococcus aureus* | 687 |
| 25 | | *Pseudomonas aeruginosa* | 359 |

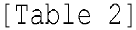

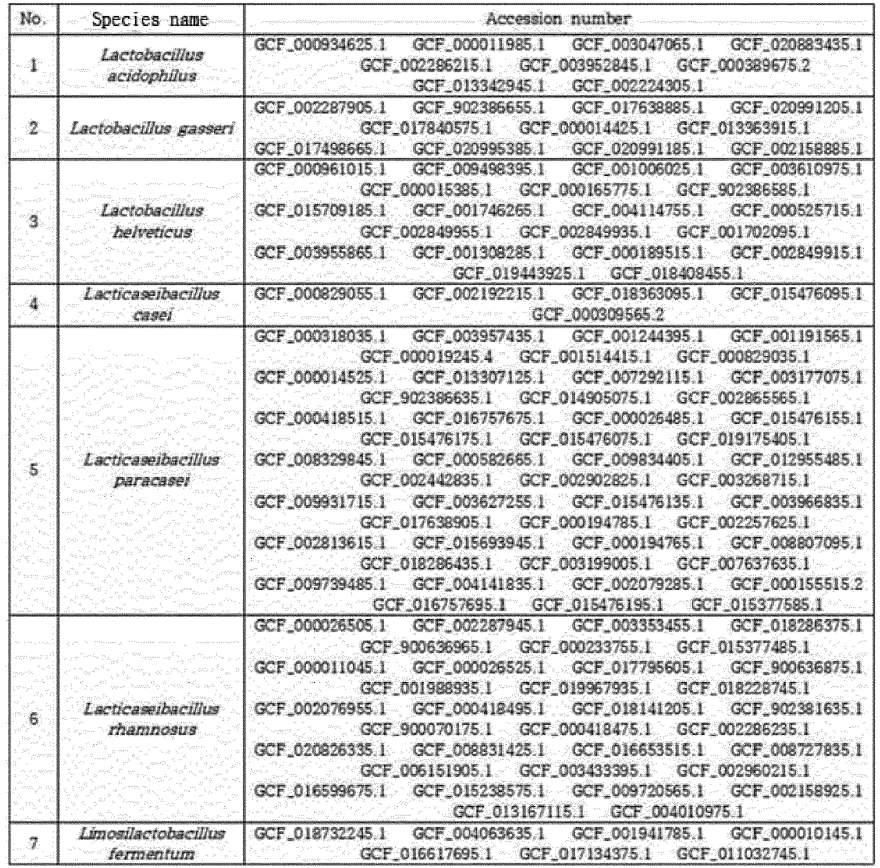

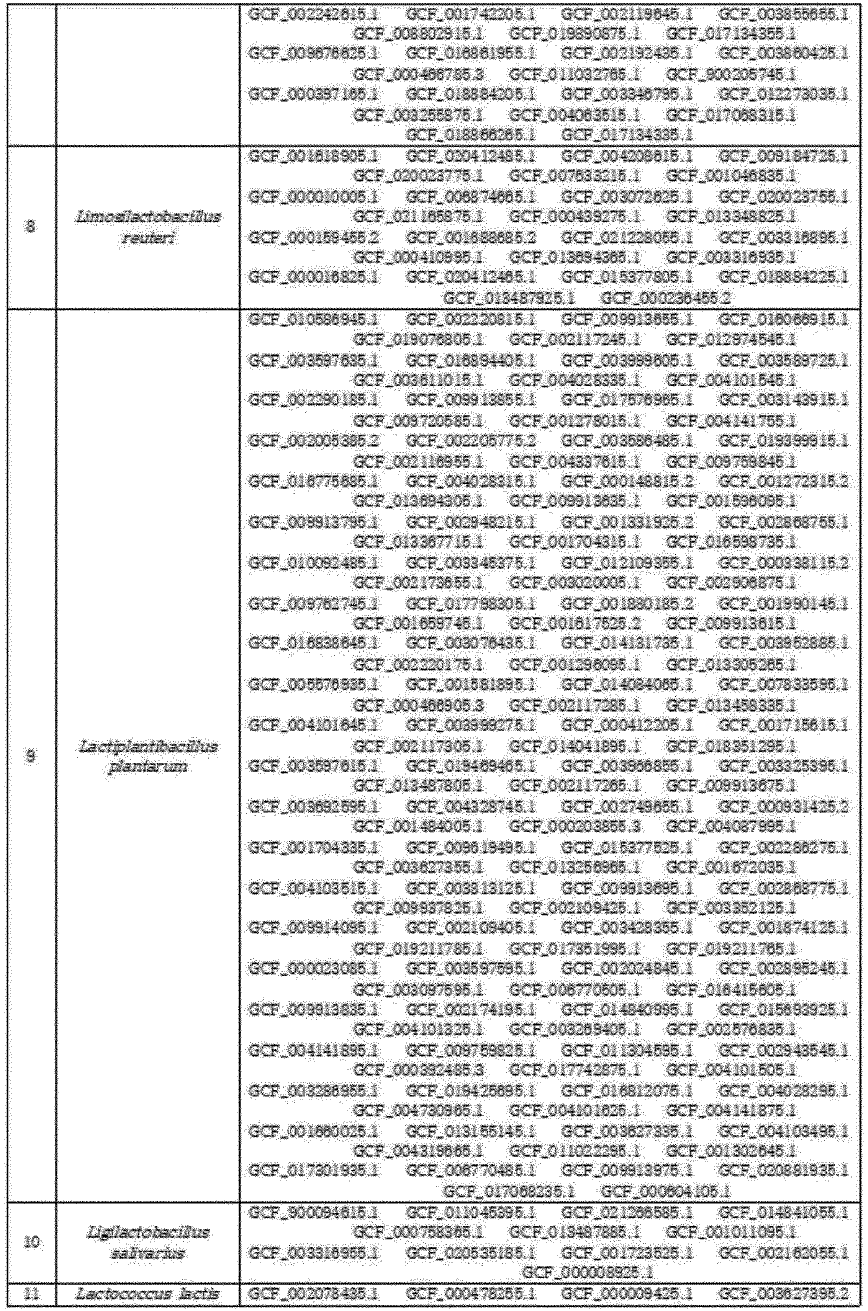

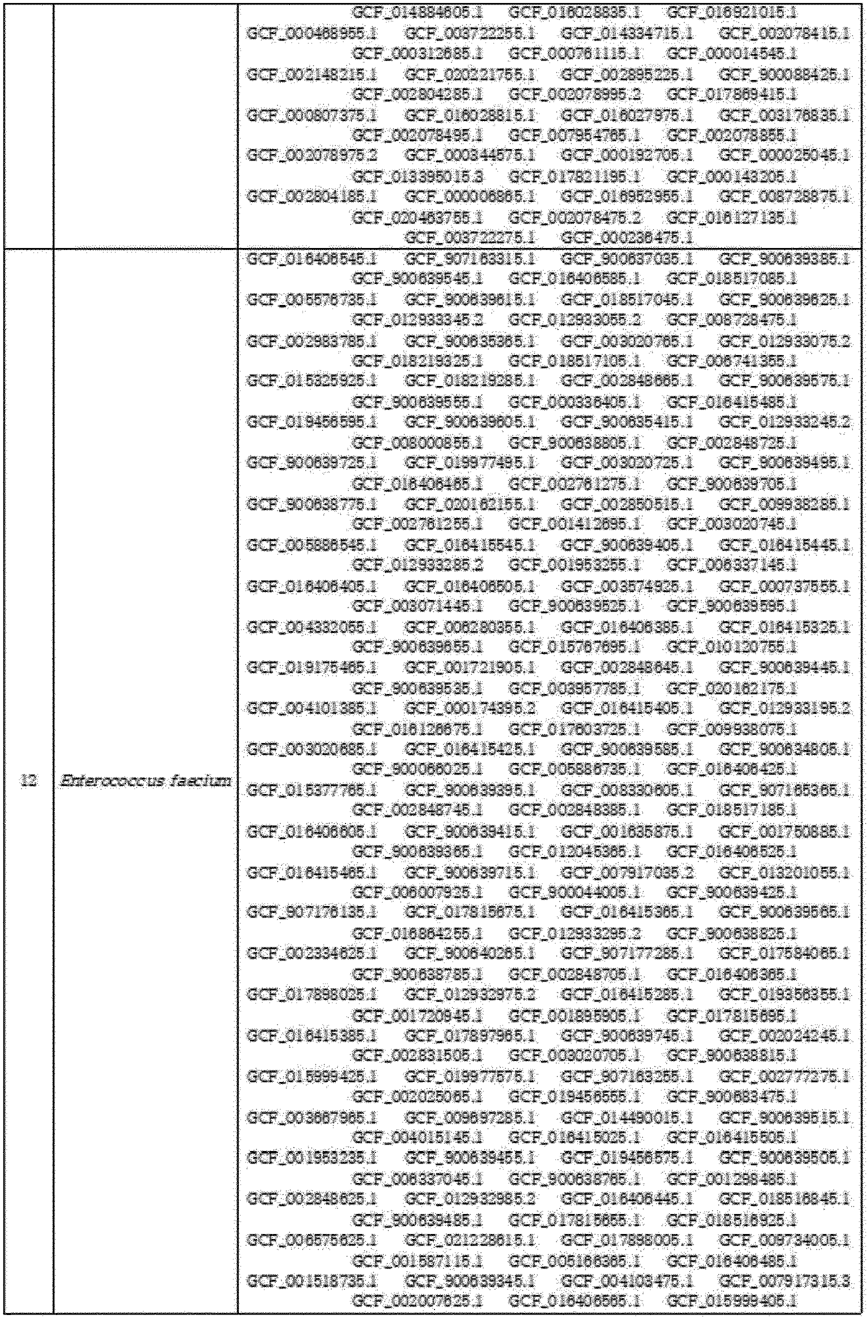

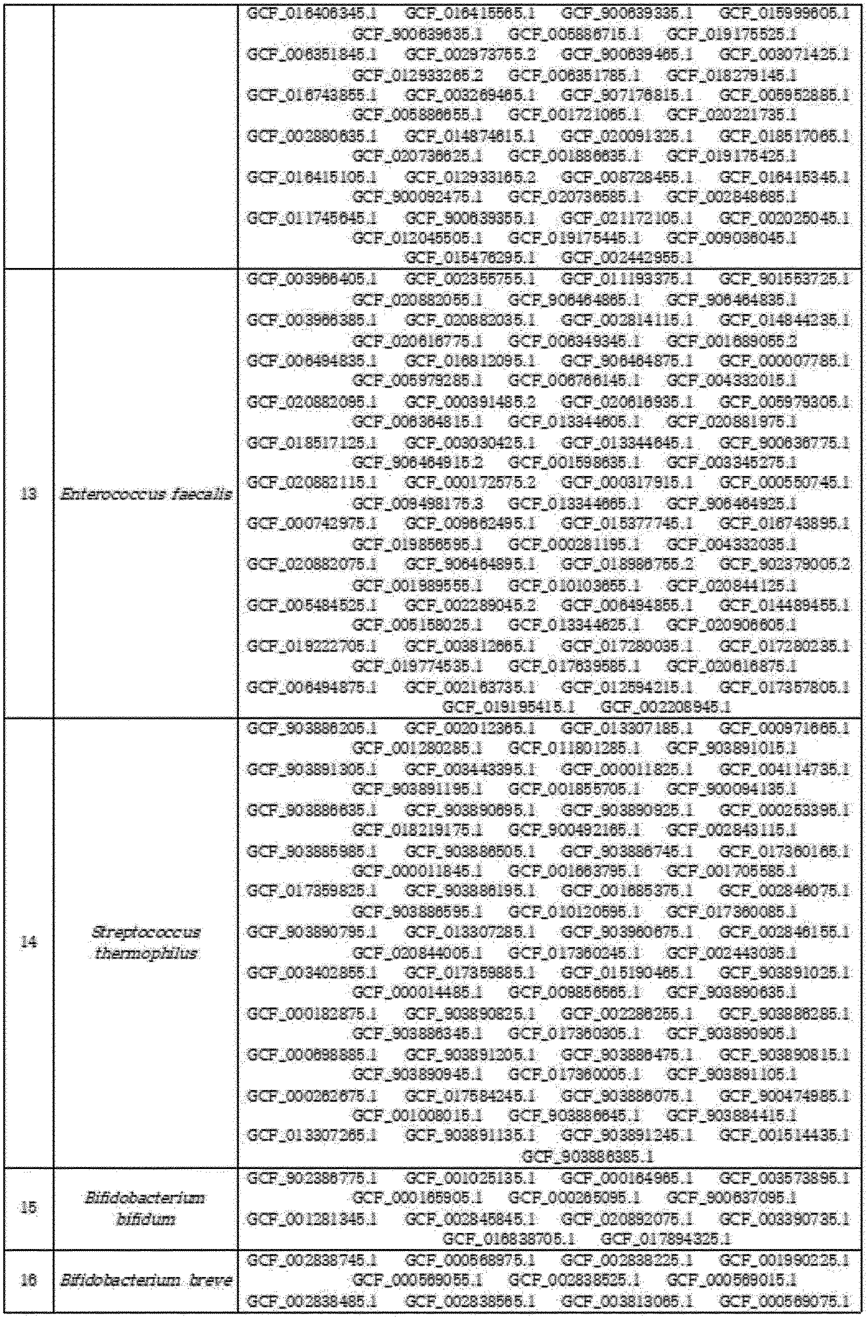

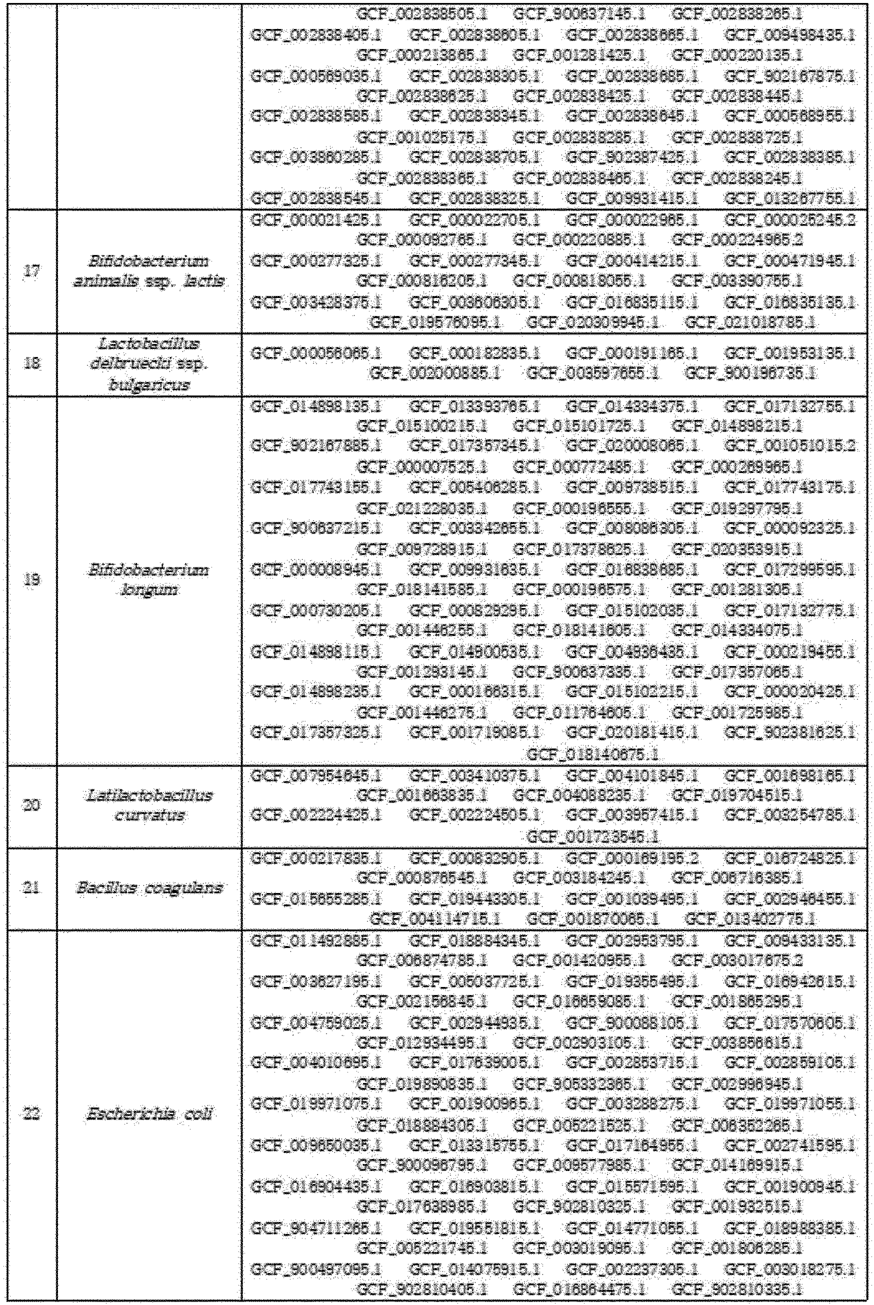

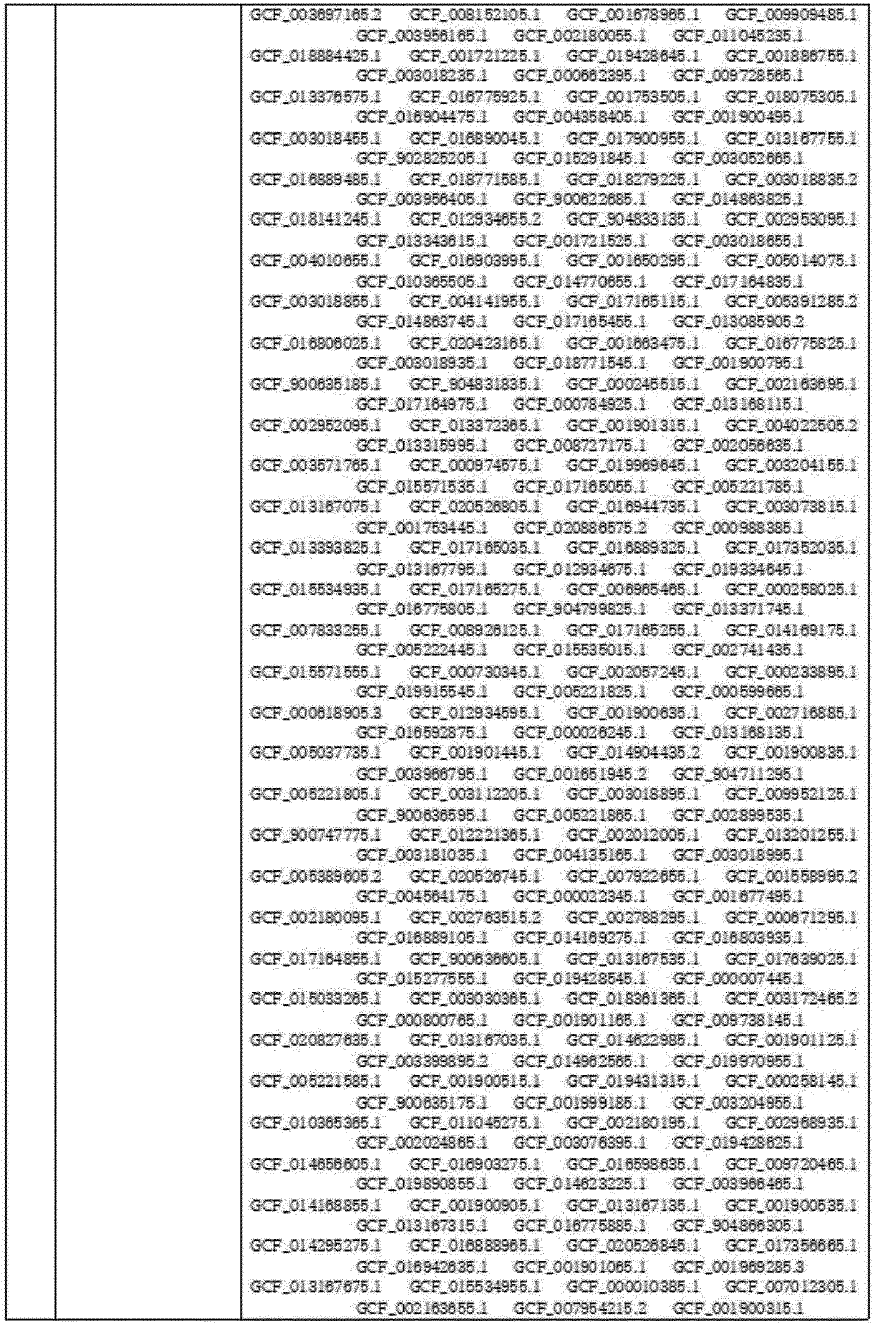

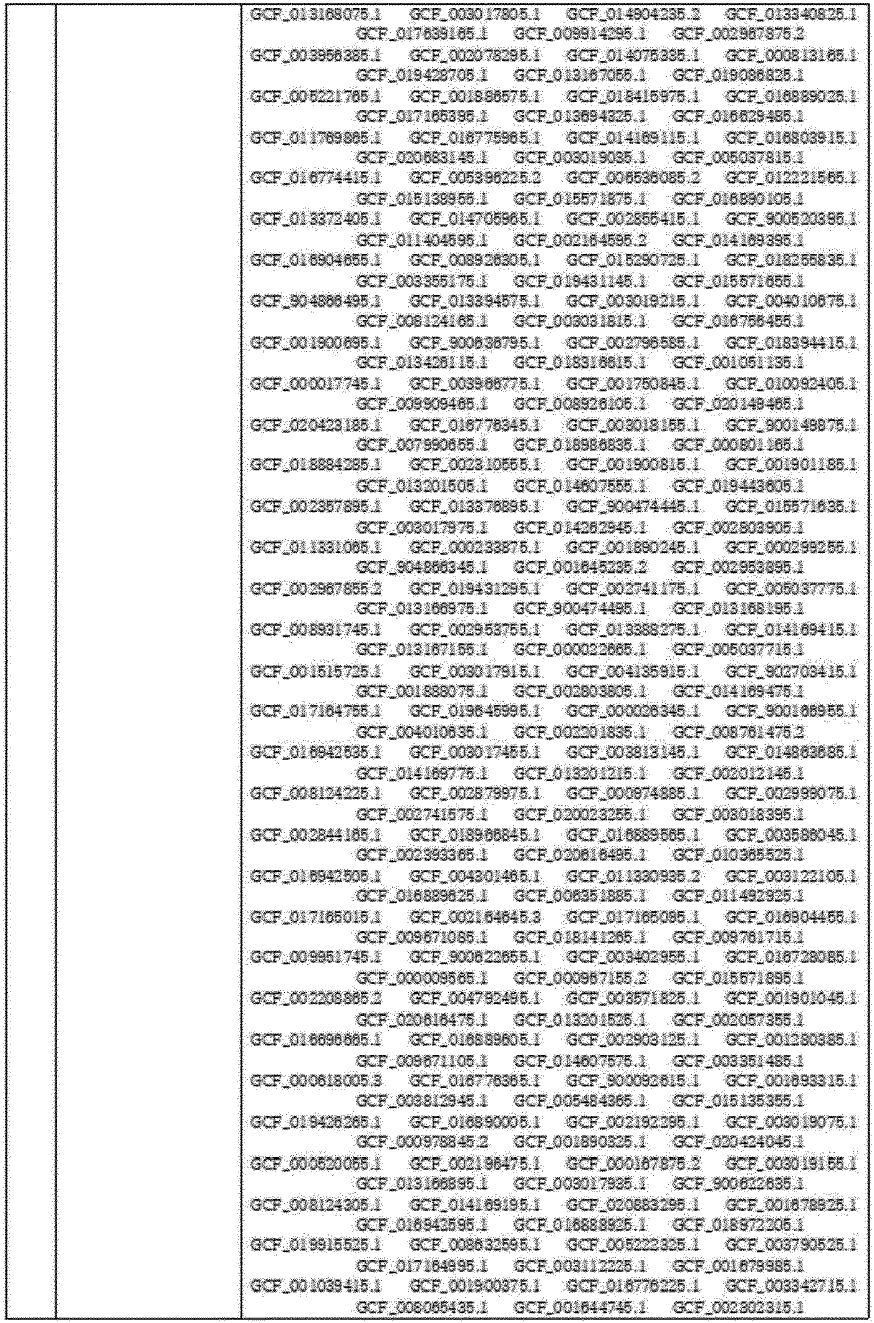

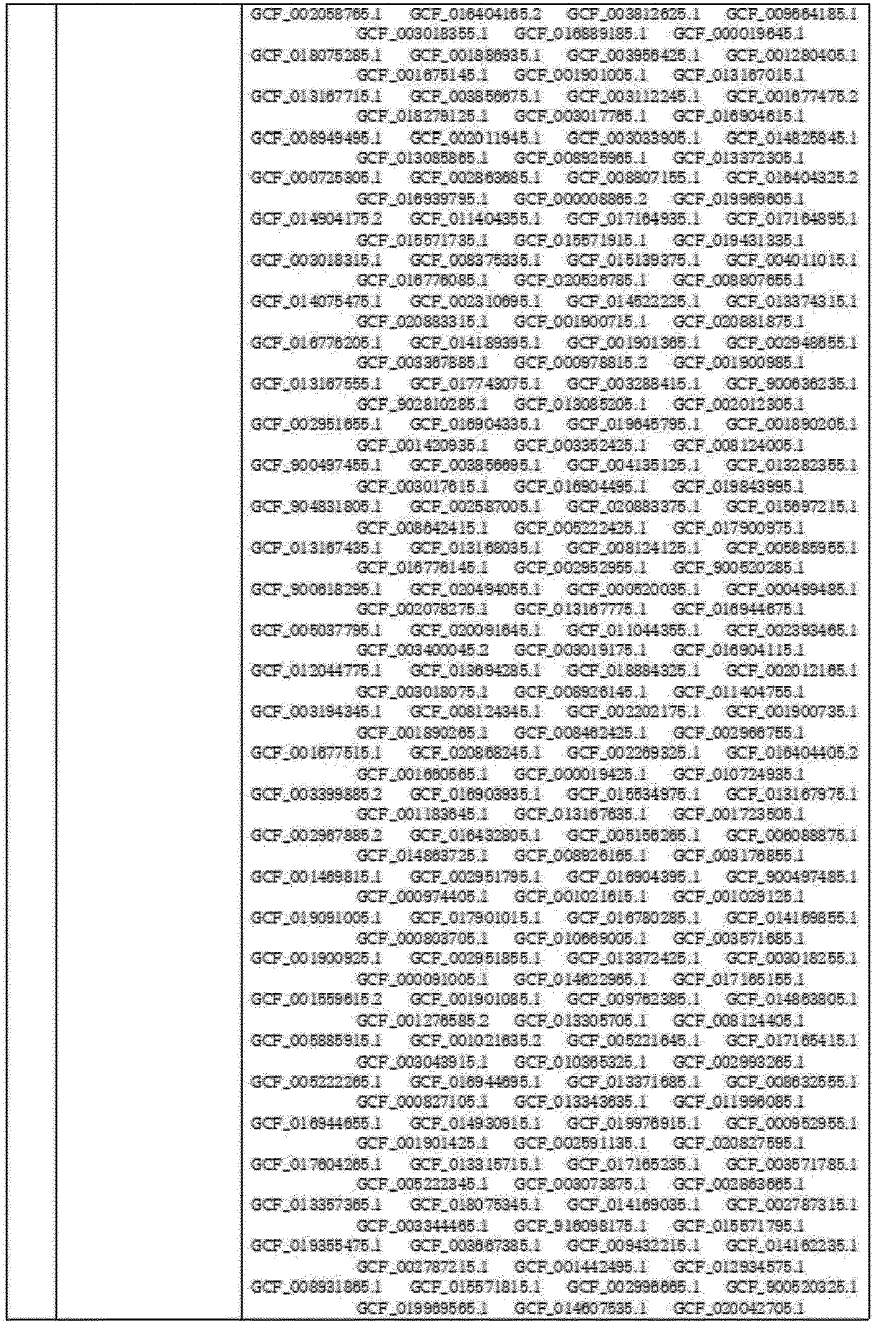

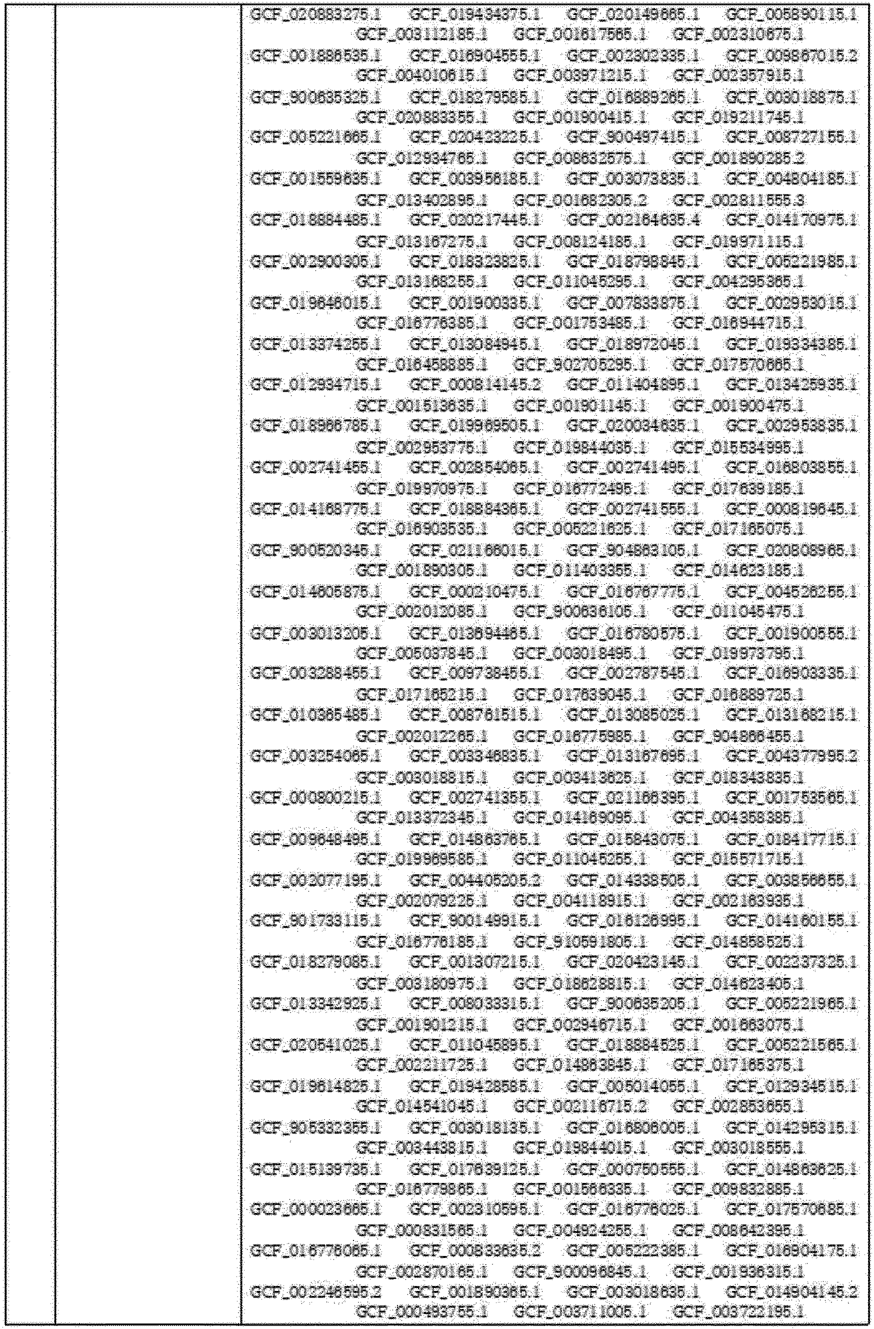

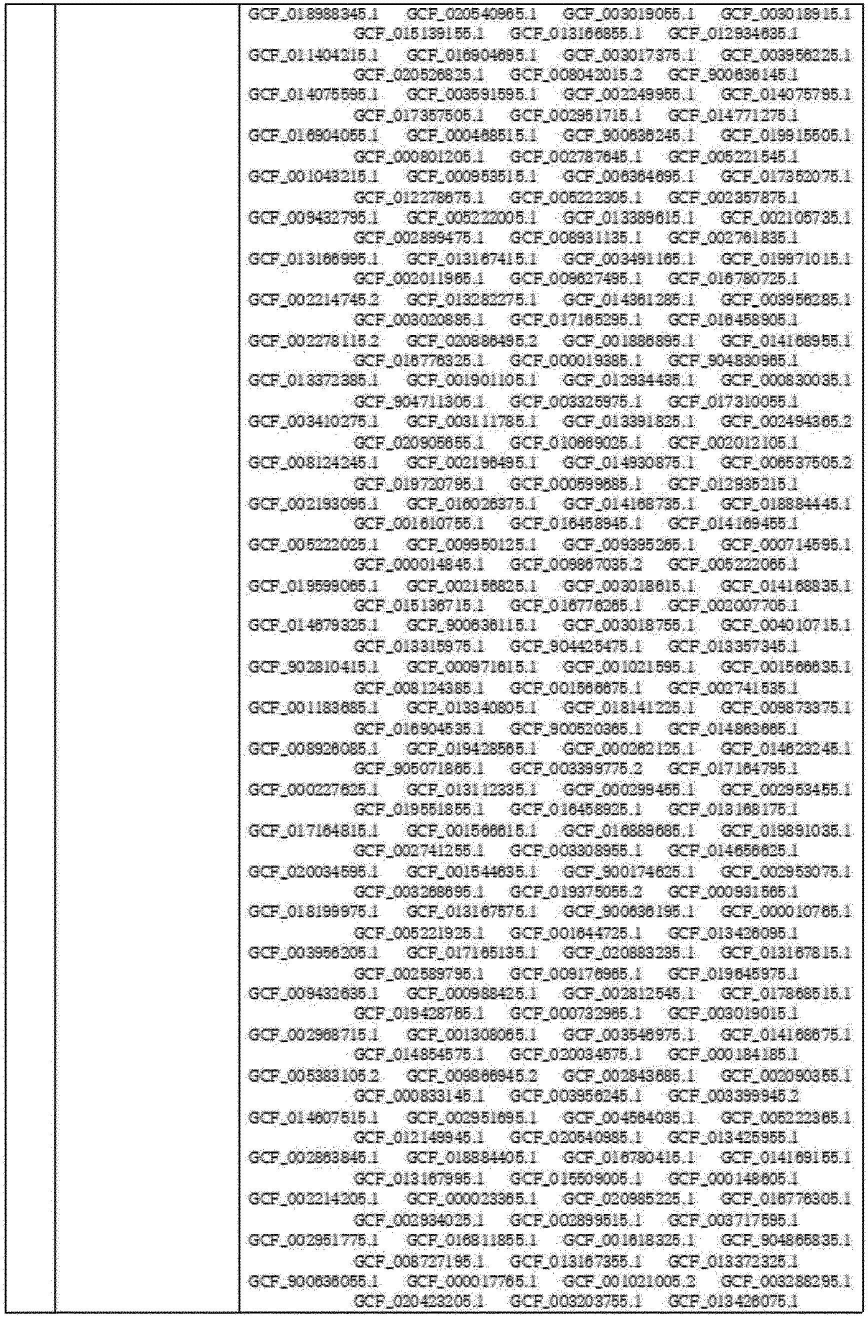

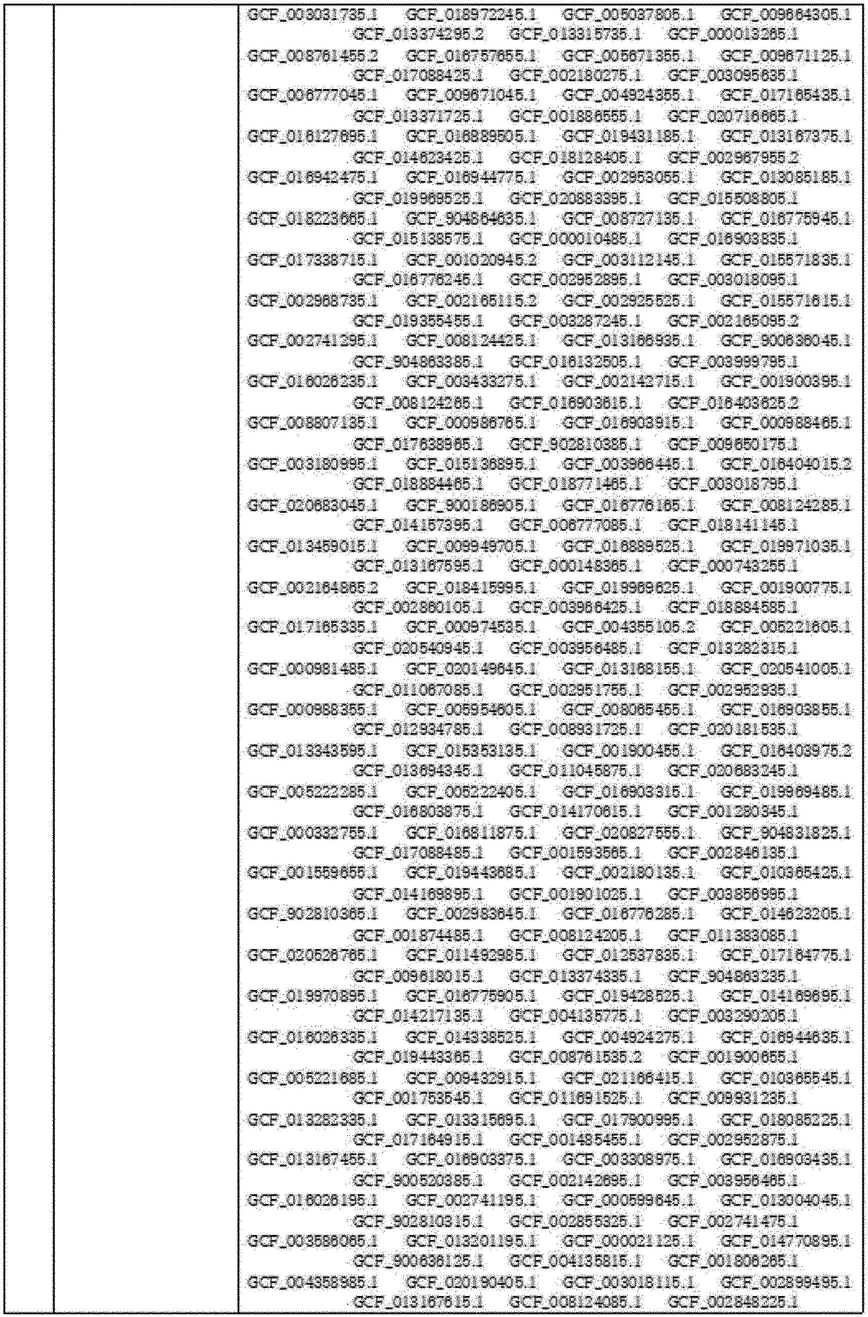

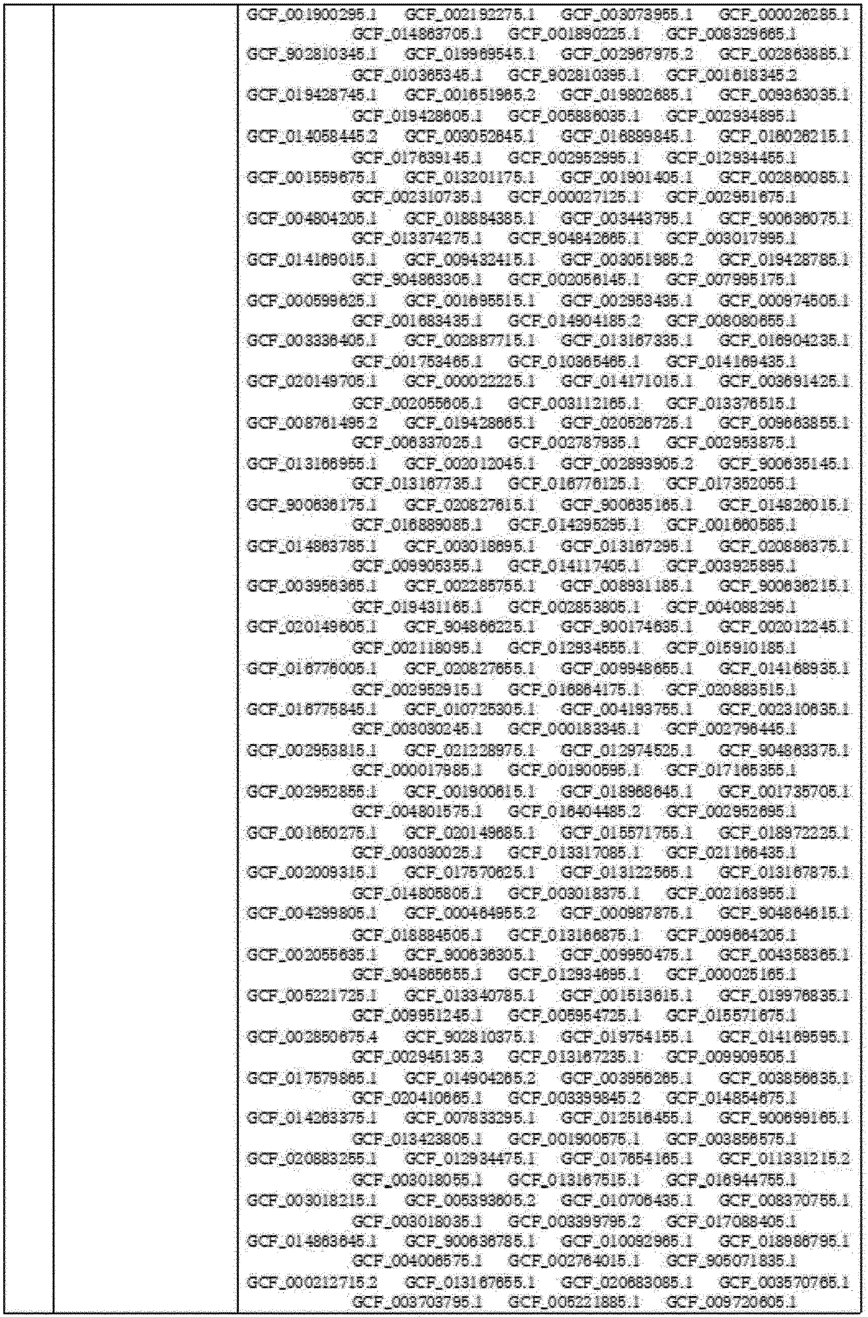

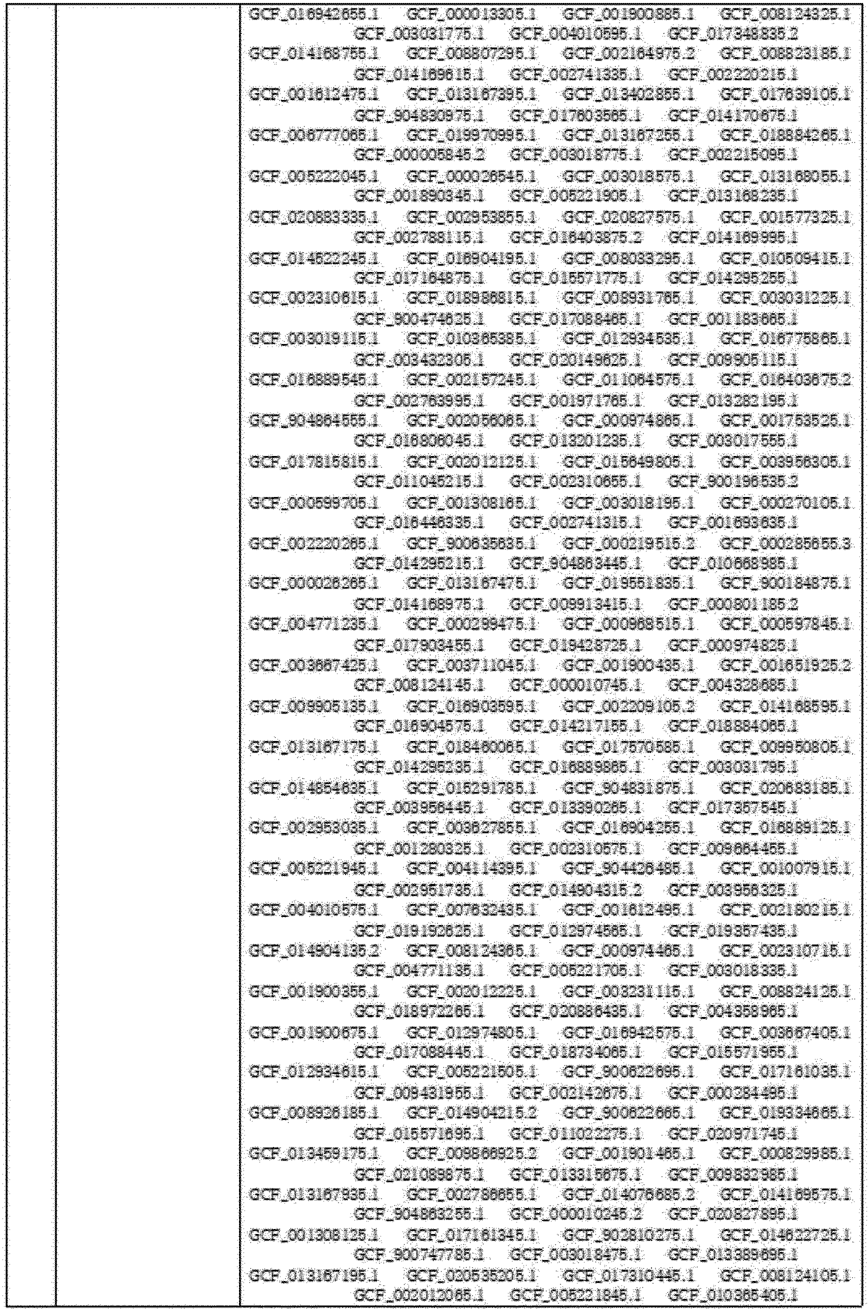

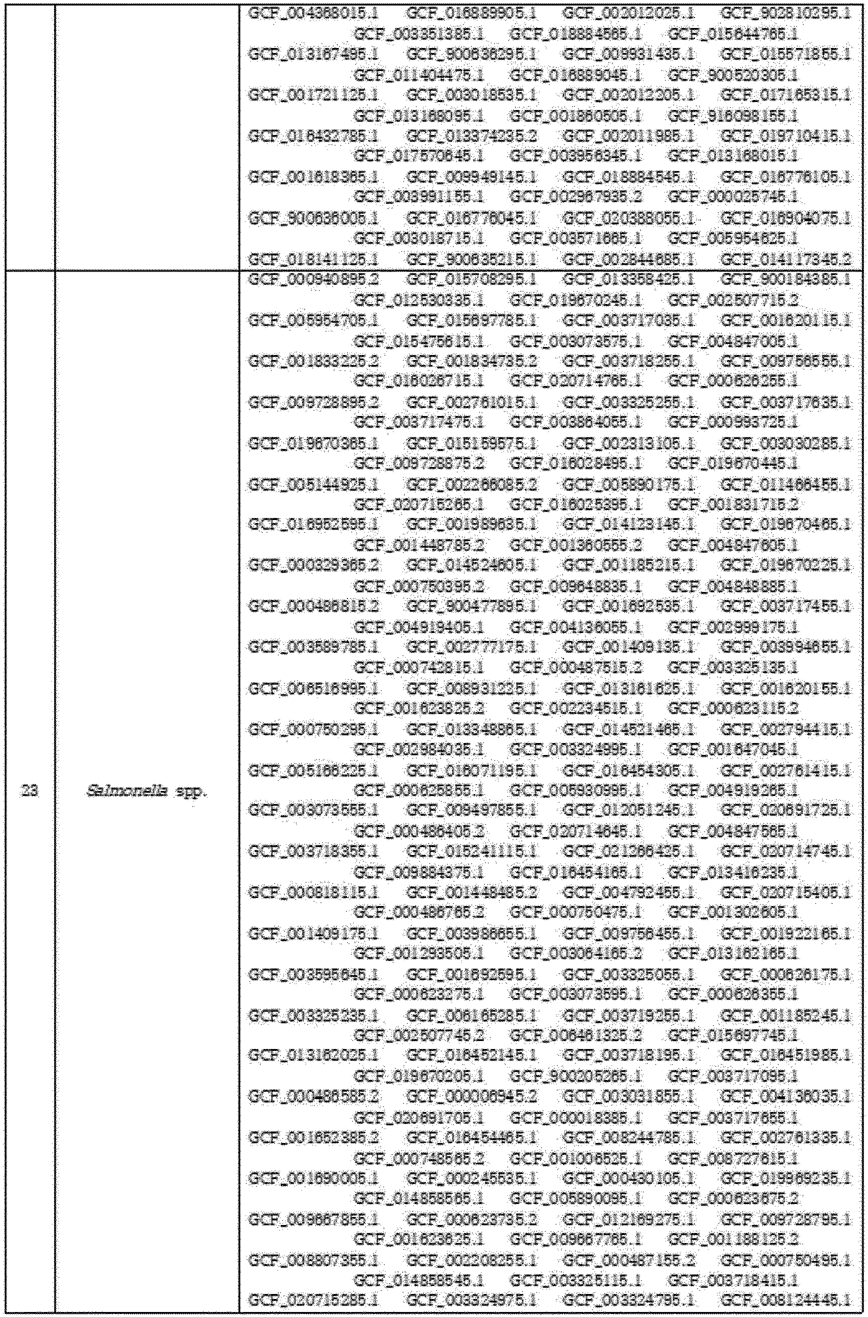

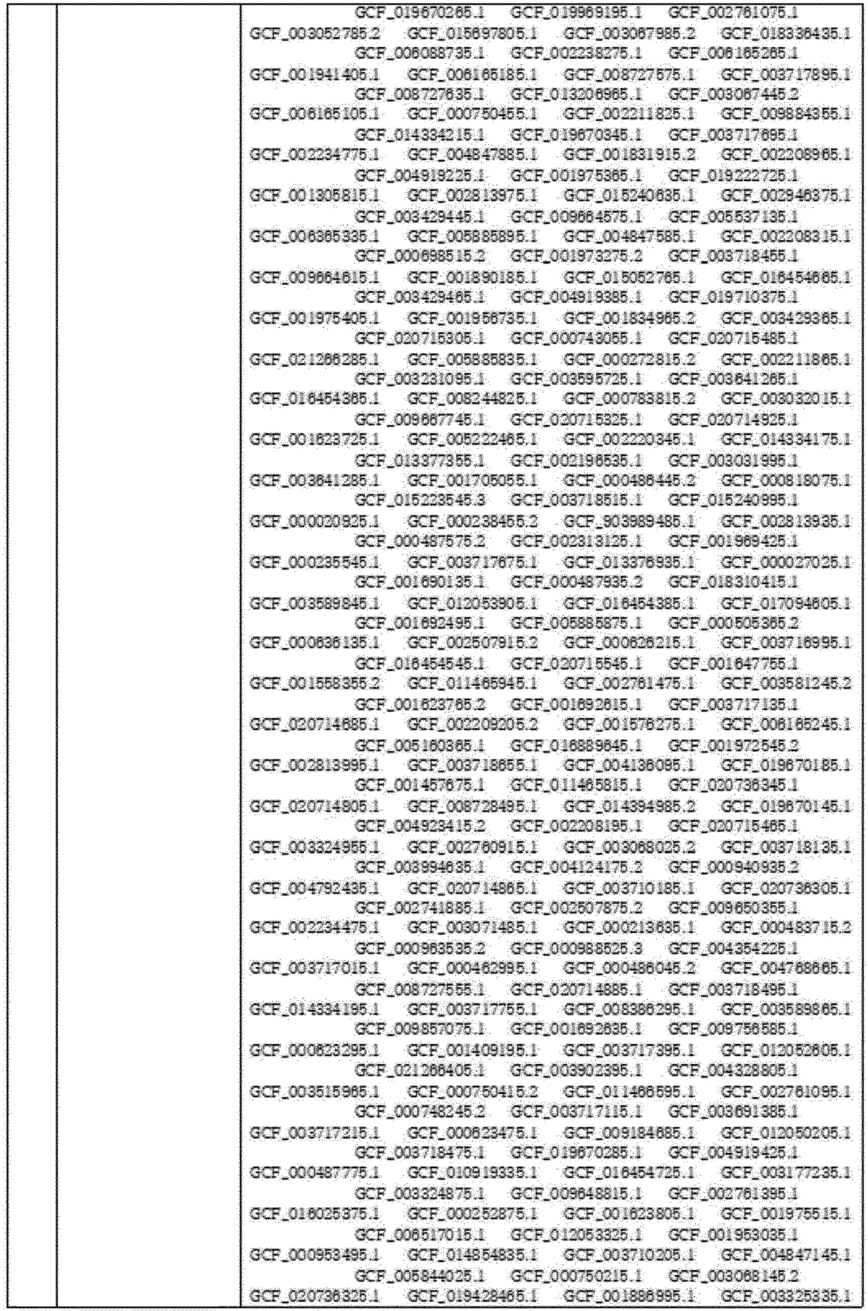

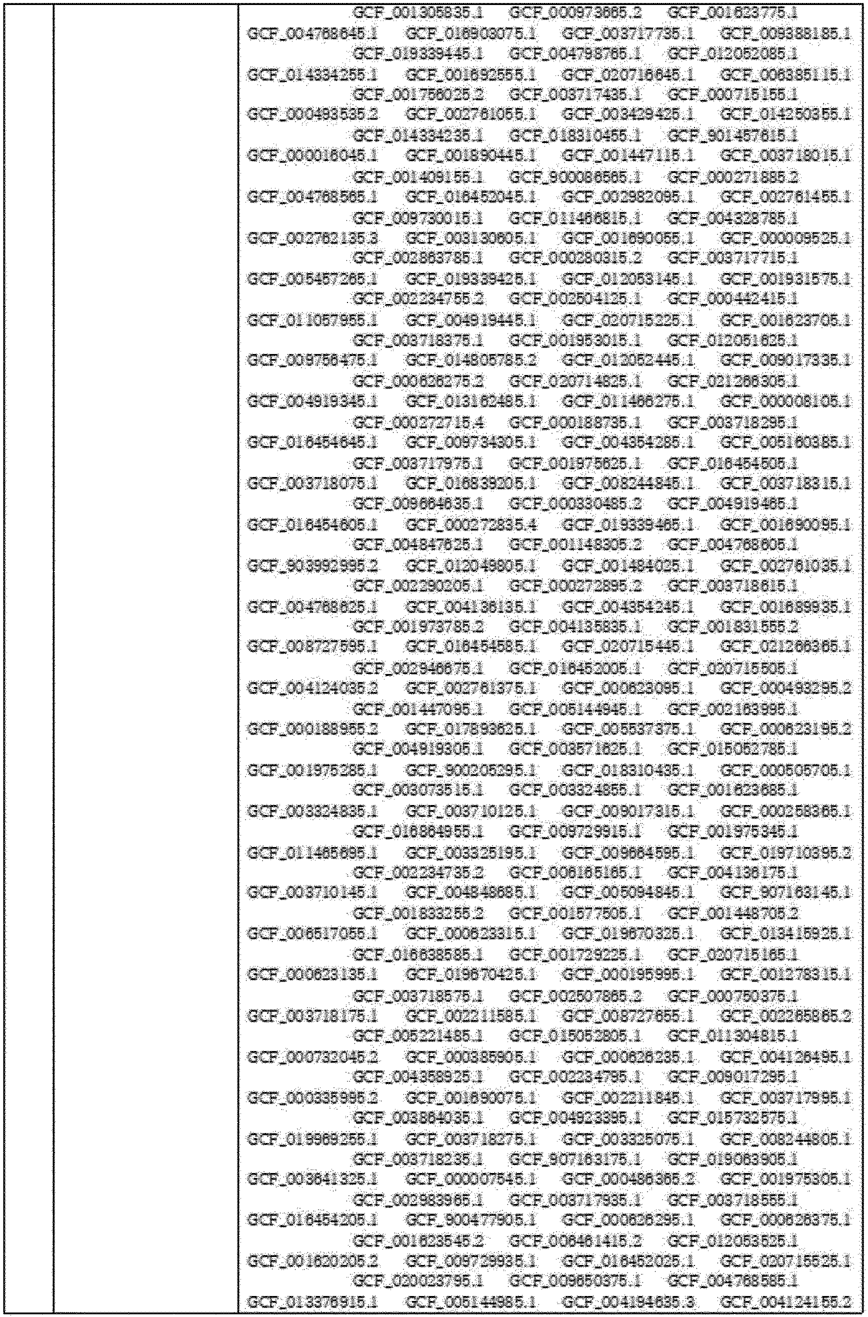

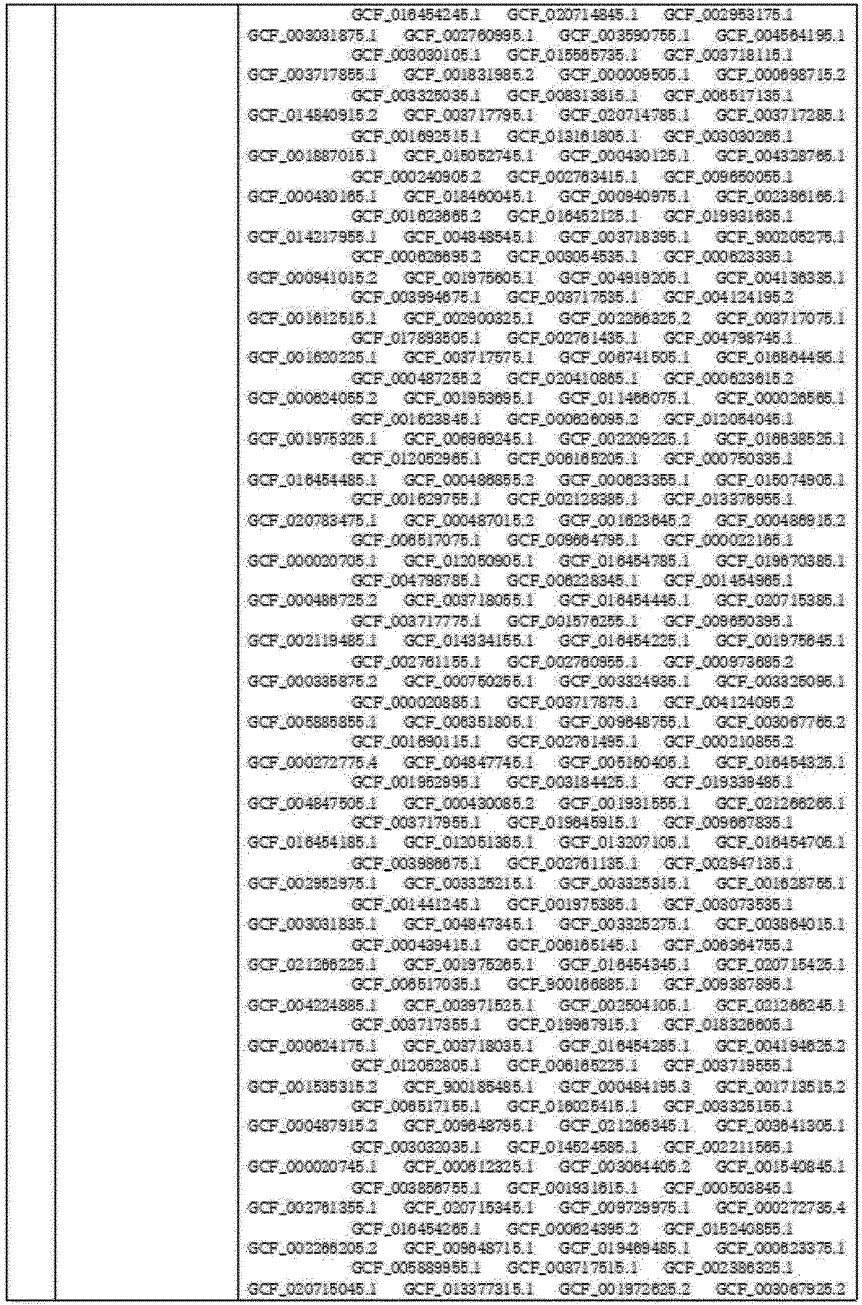

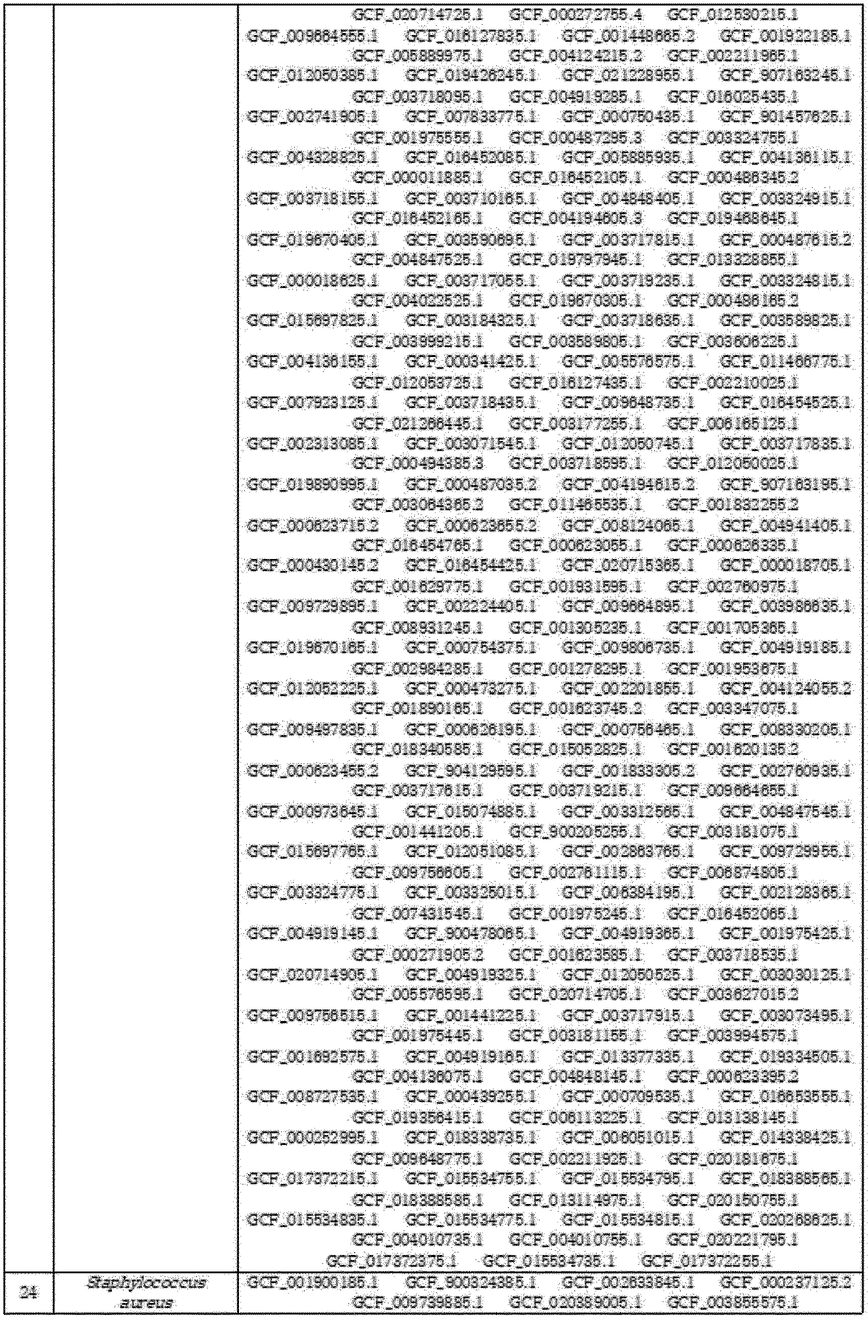

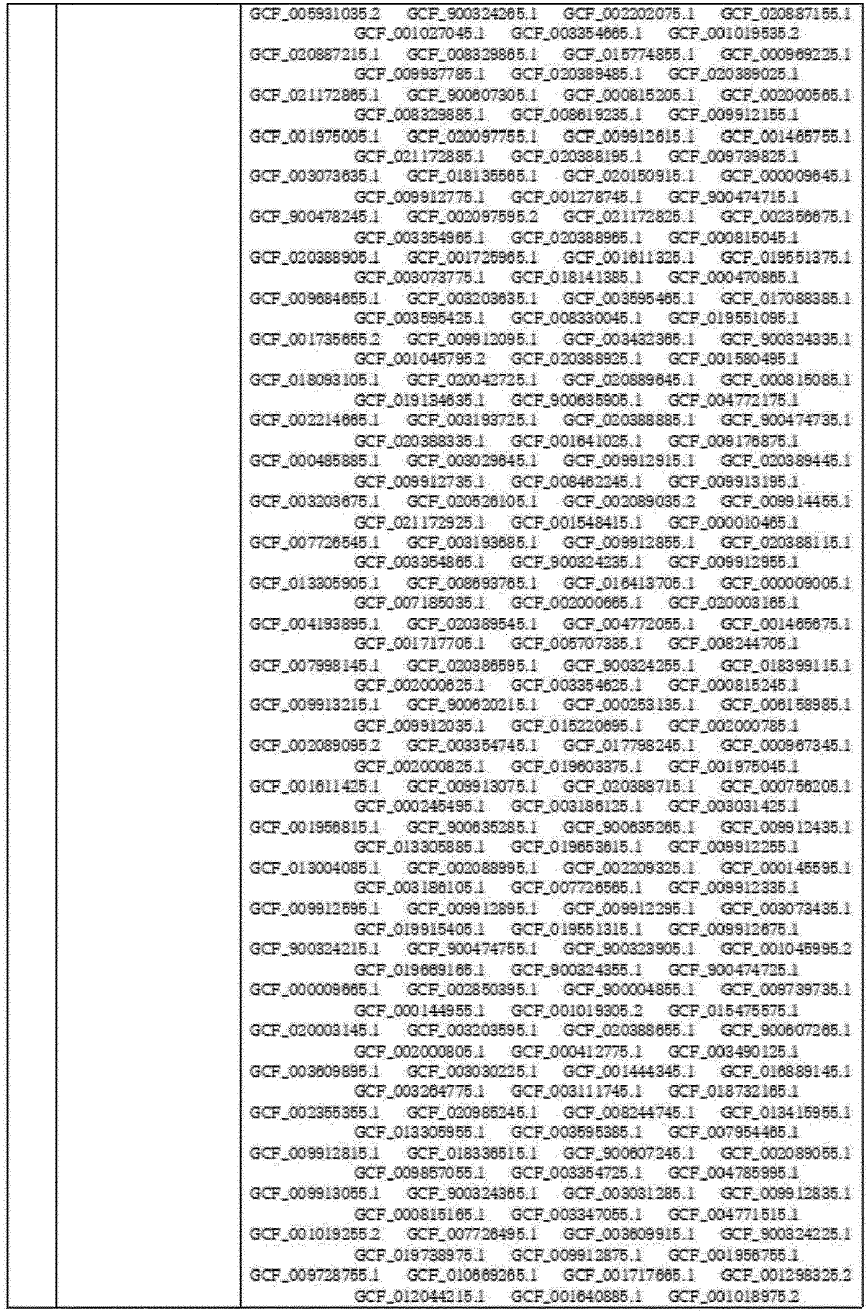

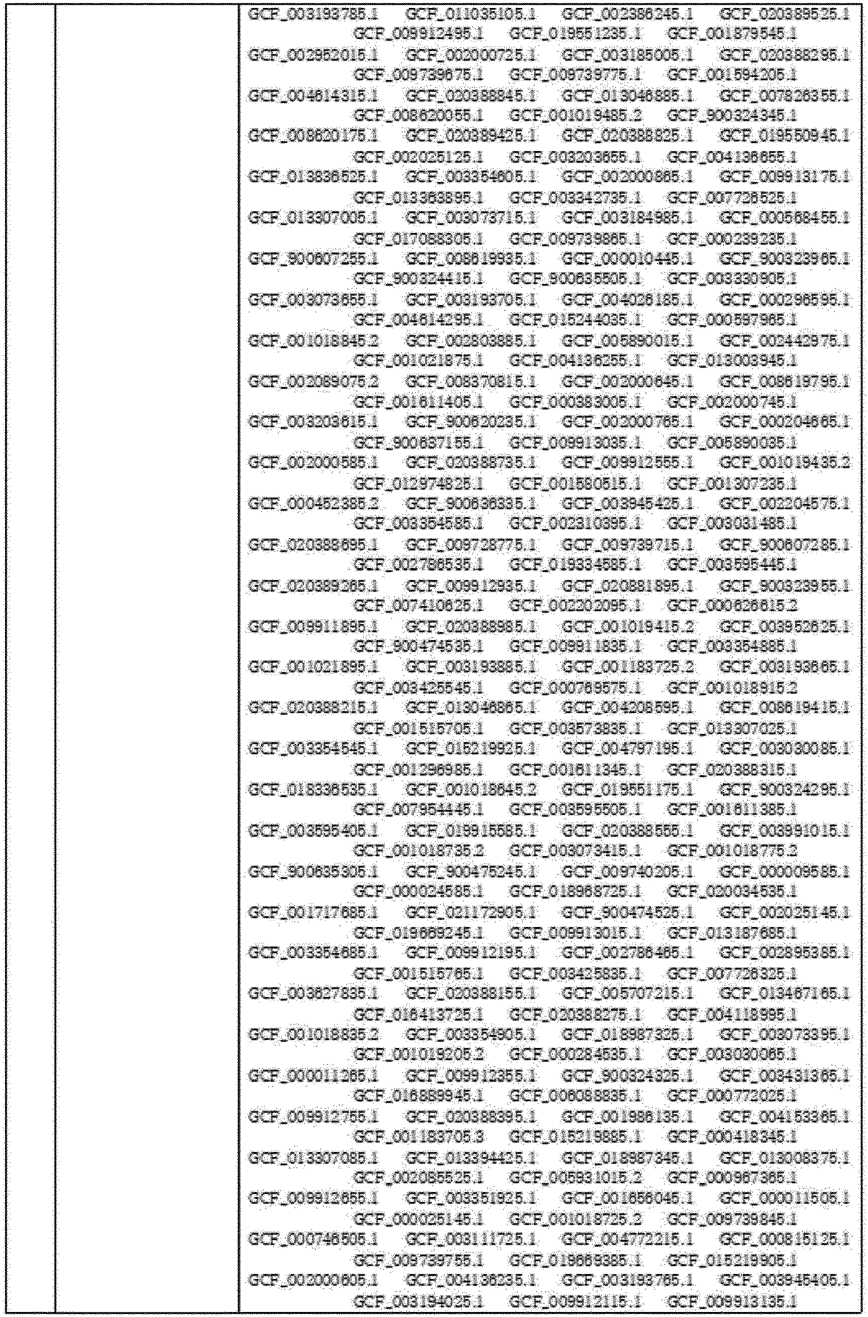

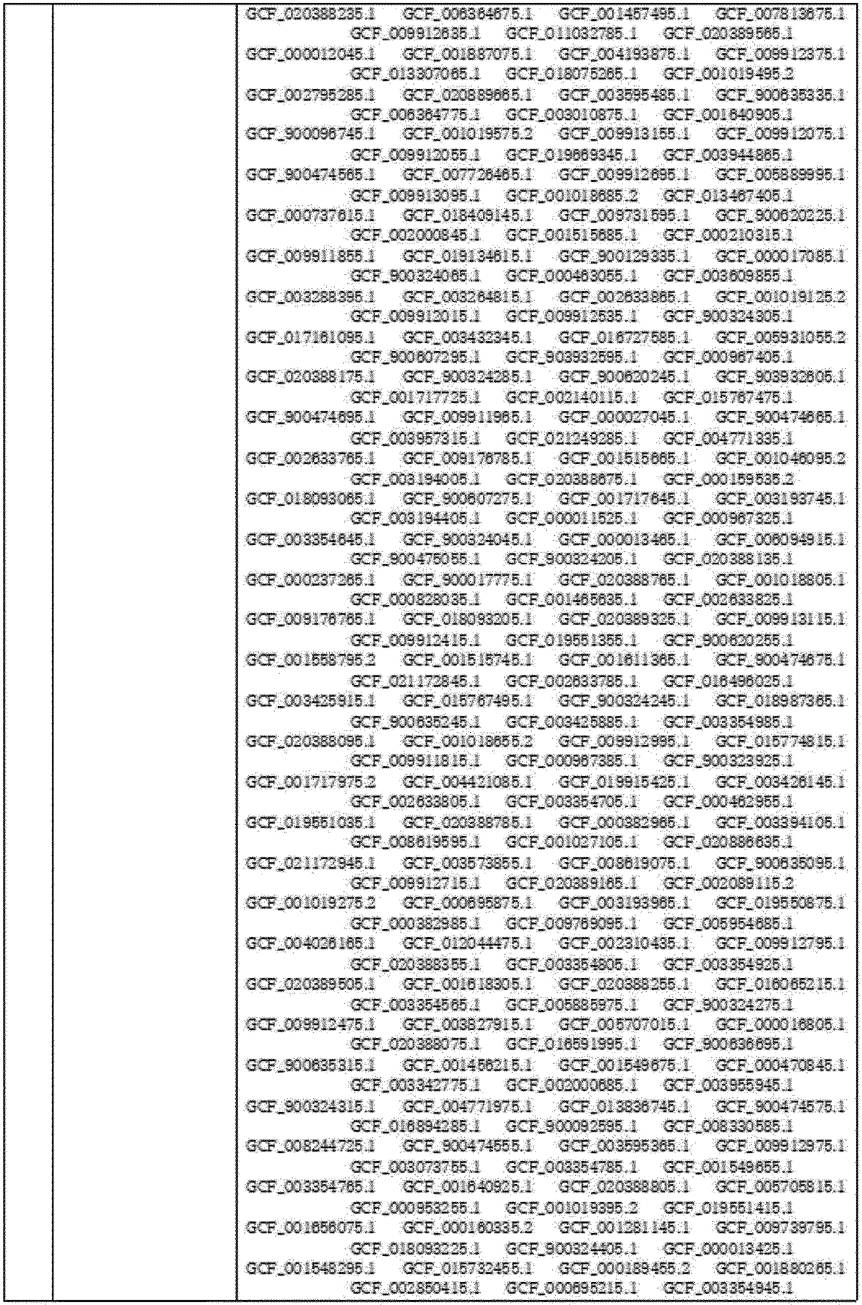

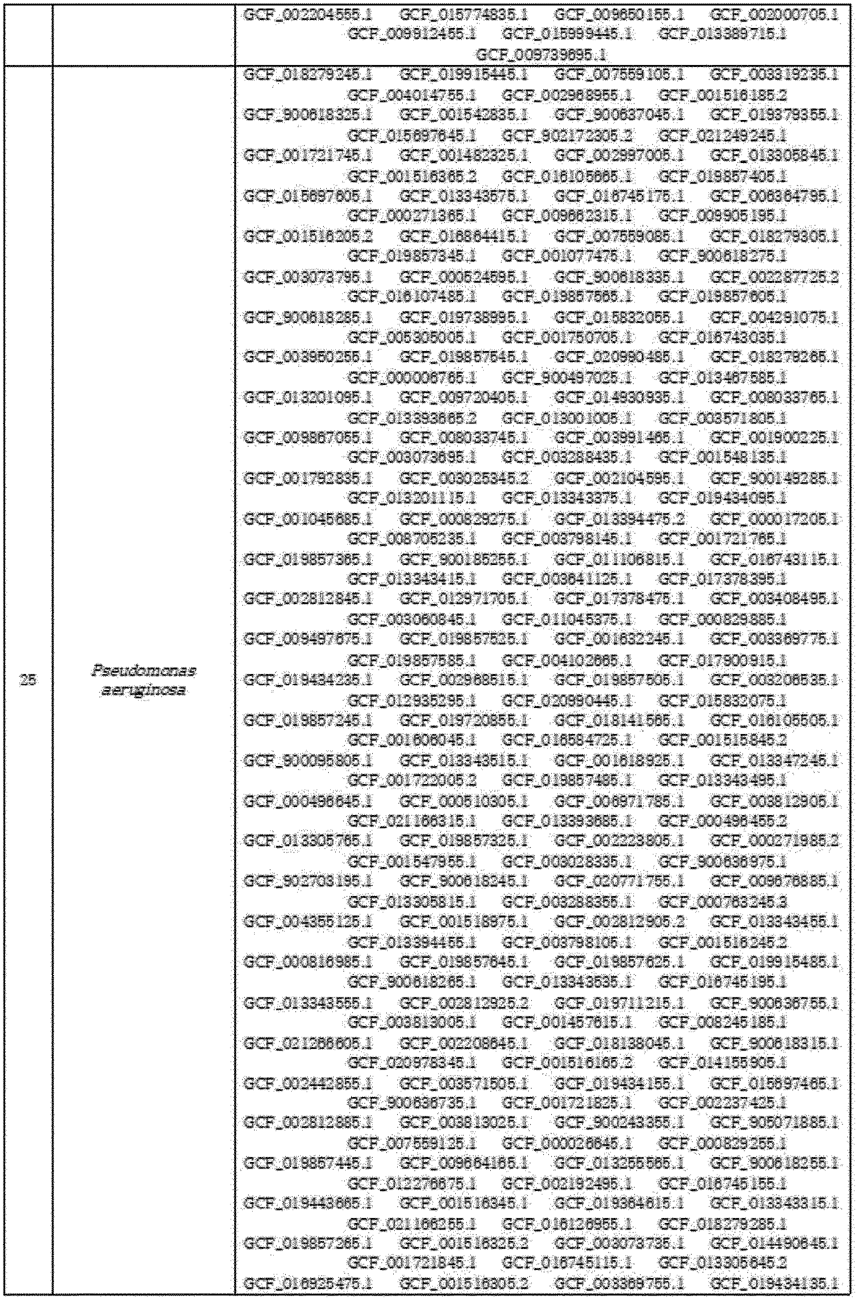

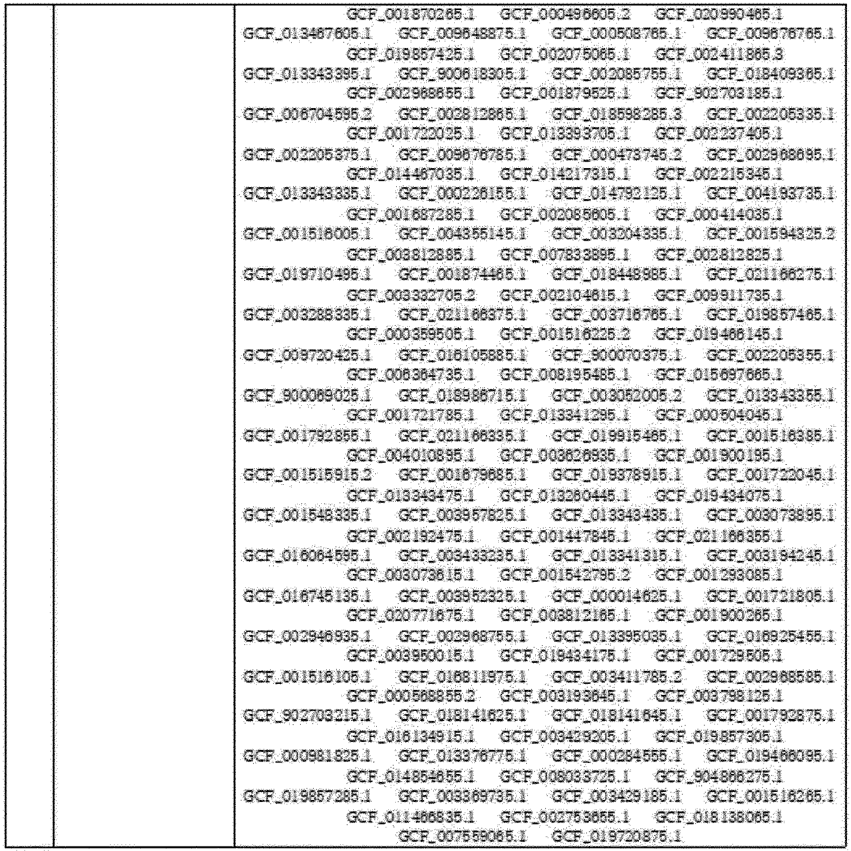

Next, the sequences of the internal transcribed spacer (ITS) regions located between the 16S and 23S rRNA of the 25 strains were acquired through in silico PCR using a consensus sequence located within about 100 bp of the latter half of 16S and a consensus sequence located within about 100 bp of the first half of 23S *(*Massimiliano et al., Appl. Environ. Microbiol., 2004, 70, 6147-6156). Using the acquired sequences, an ITS bacterial species discrimination database for metagenomic analysis was created according to the procedures of the CLC Microbial Genomics Module of CLC Genomics Workbench (Qiagen), and primer sets shown in [Table 3] below were derived. As shown in [Table 4] below, an illumina adapter sequence was designed in front of each primer sequence, and the primer set was constructed in a fusion form that amplified the target sequence as well as allowed for the construction of an NGS library. The derived primer sets were commissioned and constructed by Macrogen.

**[Table 3]**

| No. | Primer name | Sequence (5'-3') | SEQ ID NO: |
|---|---|---|---|
| 1 | ITS forward primer | TGCGGYTGGATCMCCTCCTT | 1 |
| 2 | ITS-Lactobacillus reverse primer | TCCTTCATCGRCTCCTAG | 2 |
| 3 | ITS-L.acidophilus reverse primer | CGCCCTTYTYYACTTGACC | 3 |
| 4 | ITS-Bifidobacterium reverse primer | CAGTTCTCAARCCACCAC | 4 |
| 5 | ITS-coccus reverse primer | CAATGGAGCCTAGCGGGATC | 5 |
| 6 | ITS-Enterococcus reverse primer | ATTCAACGCGGTGTTCTC | 6 |
| 7 | ITS-Enterobacteria reverse primer | CAATTTTCAGCTTGATCCAG | 7 |
| 8 | ITS-Staphylococcus reverse primer | GTTCTTYCGAACACTAGCGA | 8 |
| 9 | ITS-Pseudomonas reverse primer | GGAGGCTCGAACTCCTGACC | 9 |

**[Table 4]**

| No. | Primer name | Sequence | SEQ ID NO: |
|---|---|---|---|
| 1 | ITS forward illumine adaptor fusion primer | TCGTCGGCAGCGTCAGATGTGTATAAGA GACAGTGCGGYTGGATCMCCTCCTT | 10 |
| 2 | ITS-Lactobacillus reverse illumine adaptor fusion primer | GTCTCGTGGGCTCGGAGATGTGTATAAG AGACAGTCCTTCATGRCTCCTAG | 11 |
| 3 | ITS-L.acidophilus reverse illumine adaptor fusion primer | GTCTCGTGGGCTCGGAGATGTGTATAAG AGACAGCGCCCTTYTYYACTTGACC | 12 |
| 4 | ITS-Bifidobacterium reverse illumine adaptor fusion primer | GTCTCGTGGGCTCGGAGATGTGTATAAG AGACAGCAGTTCTCAARCCACCAC | 13 |
| 5 | ITS-coccus reverse illumine adaptor fusion primer | GTCTCGTGGGCTCGGAGATGTGTATAAG AGACAGCAATGGAGCCTAGCGGGATC | 14 |
| 6 | ITS-Enterococcus | GTCTCGTGGGCTCGGAGATGTGTATAAG | 15 |
| | reverse illumine adaptor fusion primer | AGACAGATTCAACGCGGTGTTCTC | |
| 7 | ITS-Enterobacteria reverse illumine adaptor fusion primer | GTCTCGTGGGCTCGGAGATGTGTATAAG AGACAGCAATTTTCAGCTTGATCCAG | 16 |
| 8 | ITS-Staphylococcus reverse illumine adaptor fusion primer | GTCTCGTGGGCTCGGAGATGTGTATAAG AGACAGGTTCTTYCGAACACTAGCGA | 17 |
| 9 | ITS-Pseudomonas reverse illumine adaptor fusion primer | GTCTCGTGGGCTCGGAGATGTGTATAAG AGACAGGGAGGATCGAACTCCTGACC | 18 |

### <Example 2> Establishment of ANI standards for confirmation of sequence similarity between 19 species notified by Ministry of Food and Drug Safety and identification

The program pyani for determining ANI was used in order to confirm the ANI (average nucleotide identity) between the ITS region sequences of 19 species notified by the Ministry of Food and Drug Safety acquired in <Example 1> (command to create the ANI result folder [ANI result] through the folder [19 species fastq] where the sequence information of 19 species was stored: average_nucleotide_identity.py -i [19 species fasta] -o [ANI result] -m ANIb -g). At this time, the maximum value of ANI for each strain between species was determined for the 160 bp region of the ITS sequence located after the primer region, and the ANI standard for species discrimination was set. Specifically, the maximum ANI values between species are as shown in [Table 5] below.

**[Table 5]**

| No. | Species name | Maximum ANI with other species | |
|---|---|---|---|
| | | Species name | ANI (%) |
| 1 | *Lactobacillus acidophilus* | *Lactobacillus helveticus* | 96.10 |
| 2 | *Lactobacillus gasseri* | *Lactobacillus acidophilus* | 92.20 |
| 3 | *Lactobacillus helveticus* | *Lactobacillus acidophilus* | 96.10 |
| 4 | *Lacticaseibacillus casei* | *Lacticaseibacillus paracasei* | 100 |
| 5 | *Lacticaseibacillus* | *Lacticaseibacillus casei* | 100 |
| | *paracasei* | | |
| 6 | *Lacticaseibacillus rhamnosus* | *Lacticaseibacillus casei* | 99.38 |
| 7 | *Limosilactobacillus fermentum* | *Limosilactobacillus reuteri* | 83.95 |
| 8 | *Limosilactobacillus reuteri* | *Limosilactobacillus fermentum* | 83.95 |
| 9 | *Lactiplantibacillus plantarum* | *Limosilactobacillus reuteri* | 78.86 |
| 10 | *Ligilactobacillus salivarius* | - | - |
| 11 | *Lactococcus lactis* | *Enterococcus faecalis* | 77.58 |
| 12 | *Enterococcus faecium* | *Enterococcus faecalis* | 95.30 |
| 13 | *Enterococcus faecalis* | *Enterococcus faecium* | 95.30 |
| 14 | *Streptococcus thermophilus* | *Enterococcus faecalis* | 78.21 |
| 15 | *Bifidobacterium bifidum* | *Bifidobacterium longum* | 75.19 |
| 16 | *Bifidobacterium breve* | *Bifidobacterium longum* | 91.25 |
| 17 | *Bifidobacterium animalis ssp. lactis* | - | - |
| 18 | *Lactobacillus delbruecki ssp. Bulgaricus* | *Lactobacillus helveticus* | 77.78 |
| 19 | *Bifidobacterium longum* | *Bifidobacterium breve* | 91.25 |

In the analysis results, - means that the ANI value is 0% as the calculation results with any species, and other than that, when the ANI with another species is the maximum value, the ANI output value and the corresponding species name are described.

As a result, as shown in Table 5, it has been confirmed that the ANI of the ITS region sequence was 99% or more in some strains of *Lacticaseibacillus casei, Lacticaseibacillus paracasei,* and *Lacticaseibacillus rhamnosus,* which belong to the genus *Lacticaseibacillus,* and the ANI was less than 97% for all other species.

Based on the above results, it has been confirmed that probiotic species can be discriminated as the genus *Lacticaseibacillus* and the remaining 17 species through the discrimination of 19 probiotic species notified by the Ministry of Food and Drug Safety. Specifically, the procedures of the CLC Microbial Genomics Module of CLC Genomics Workbench (Qiagen) were followed for species discrimination, and the ITS region sequence acquired in <Example 1> was used as the reference sequence for discrimination. The ANI for discrimination was set to 98% or more so that only sequences discriminated as *Lacticaseibacillus casei, Lacticaseibacillus paracasei,* or *Lacticaseibacillus rhamnosus* were discriminated as the genus *Lacticaseibacillus* and the remaining 17 probiotic species were each discriminated as a single species.

### <Example 3> Homology test between whole genomes of 19 species notified by Ministry of Food and Drug Safety and all bacterial species listed in NCBI

The homology of the whole genomes of 19 species notified by the Ministry of Food and Drug Safety and all bacterial species listed in NCBI was tested by the method for examining sequence similarity between the ITS region sequences of 19 species notified by the Ministry of Food and Drug Safety acquired in <Example 1> and the bacterial species in <Example 2>.

Specifically, the whole genomes of a total of 24,487 bacterial species were acquired through searching with filter conditions "Search all[filter] AND bacteria[filter] AND latest[filter] AND "complete genome"[filter] AND all[filter] NOT anomalous[filter]" in the GenBank database of the U.S. National Center for Biological Information (NCBI). Next, the sequences of all bacterial species that could be acquired from the primer sets in [Table 3] were acquired through in silico PCR using the primer sets in [Table 3]. Thereafter, the results of homologous bacterial species confirmation through ITS sequence comparison between 19 species notified by the Ministry of Food and Drug Safety and other species are shown in [Table 6]. Here, the standard for determining that bacterial species were homologous was the ANI value between sequences of 98% or more.

**[Table 6]**

| No. | Species name | Species confirmed to be homologous |
|---|---|---|
| 1 | *Lactobacillus acidophilus* | - |
| 2 | *Lactobacillus gasseri* | *Lactobacillus paragasseri* |
| 3 | *Lactobacillus helveticus* | - |
| 4 | *Lacticaseibacillus paracasei*/*casei*/*rhamnosus* | *Lacticaseibacillus zeae* |
| 5 | *Limosilactobacillus fermentum* | - |
| 6 | *Limosilactobacillus reuteri* | - |
| 7 | *Lactiplantibacillus plantarum* | *Lactiplantibacillus argentoratensis, lactiplantibacilus pentosus, lactiplantibacillus paraplantarum* |
| 8 | *Ligilactobacillus salivarius* | - |
| 9 | *Lactococcus lactis* | *Lactococus cremoris* |
| 10 | *Enterococcus faecium* | *Enterococcus lactis* |
| 11 | *Enterococcus faecalis* | *Enterococcus lactis* |
| 12 | *Streptococcus thermophilus* | *Streptococcus salivarius, Streptococus vestibularis* |
| 13 | *Bifidobacterium bifidum* | - |
| 14 | *Bifidobacterium brev* | - |
| 15 | *Bifidobacterium animalis ssp. Lactis* | - |
| 16 | *Lactobacillus delbruecki ssp. Bulgaricus* | - |
| 17 | *Bifidobacterium longum* | - |

As a result, as shown in Table 6, only 9 species among the total bacterial species listed in NCBI have been confirmed to be homologous with the 19 species notified by the Ministry of Food and Drug Safety.

### <Example 4> Discrimination of bacterial species in mixed strain suspension

It was examined whether that 19 probiotic species notified by the Ministry of Food and Drug Safety, 2 probiotic species added in food, and 4 pathogenic bacterial species in a mixed strain suspension could be discriminated by the method of the present invention.

Specifically, as shown in [Table 7] below, the standard strains of 19 probiotic species notified by the Ministry of Food and Drug Safety were acquired from the National Academy of Agricultural Sciences and the Korean Culture Center of Microorganisms, 2 probiotic species added in food were provided from HY and Sabinsa and isolated, and 4 pathogenic bacterial species were acquired from ATCC. Thereafter, discrimination of the strains in a mixed strain suspension was performed using the fusion primer sets of <Example 1>.

**[Table 7]**

| No. | Species name | Distributor | Strain number |
|---|---|---|---|
| 1 | *Lactobacillus acidophilus* | National Institute of Agricultural Sciences | KACC 12419 |
| 2 | *Lactobacillus gasseri* | | KACC 12424 |
| 3 | *Lactobacillus helveticus* | | KACC 12418 |
| 4 | *Lacticaseibacillus casei* | | KACC 12413 |
| 5 | *Lacticaseibacillus paracasei* | | KACC 12361 |
| 6 | *Lacticaseibacillus rhamnosus* | | KACC 11953 |
| 7 | *Limosilactobacillus fermentum* | | KACC 11441 |
| 8 | *Limosilactobacillus reuteri* | | KACC 11452 |
| 9 | *Lactiplantibacillus plantarum* | | KACC 11451 |
| 10 | *Ligilactobacillus salivarius* | | KACC 10006 |
| 11 | *Lactococcus lactis* | | KACC 13877 |
| 12 | *Enterococcus faecium* | | KACC 11954 |
| 13 | *Enterococcus faecalis* | | KACC 13807 |
| 14 | *Streptococcus thermophilus* | | KACC 11857 |
| 15 | *Bifidobacterium bifidum* | | KACC 20601 |
| 16 | *Bifidobacterium breve* | | KACC 16639 |
| 17 | *Bifidobacterium animalis ssp. Lactis* | | KACC 16638 |
| 18 | *Lactobacillus delbruecki ssp. Bulgaricus* | Korean Culture Center of Microorganisms | KCCM 35463 |
| 19 | *Bifidobacterium longum* | | KCCM 11953 |
| 20 | *Latilactobacillus curvatus* | HY | HY7601 |
| 21 | *Bacillus coagulans* | Sabinsa | MTCC5856 |
| 22 | *Escherichia coli* | ATCC | ATCC 8739 |
| 23 | *Salmonella spp.* | | ATCC 14028 |
| 24 | *Staphylococcus aureus* | | ATCC 6538 |
| 25 | *Pseudomonas aeruginosa* | | ATCC 9027 |

Each strain was cultured according to the method suggested by the distributor, then the number of viable bacteria was measured by the dispensing well plate method, and the strain was appropriately diluted with PBS (10⁵ to 10⁸ CFU/mL), then mixed with other strains at an appropriate ratio (0.01% to 10%), and used as a test specimen.

Next, DNA was extracted using the Omega pathogen kit (Omega, #51604) according to the manufacturer's procedures, and the extracted DNA was purified using HiAccuBead (AccuGene, #ACN01.50) according to the manufacturer's procedures.

Amplicon PCR was performed on the extracted sample DNA using the fusion primer sets of <Example 1> as follows. To the extracted sample DNA, 10 mM Tris pH 8.5 was added, dilution was performed to have a DNA concentration of 12 ng/µL, a PCR reaction solution was prepared using the fusion primer constructed in <Example 1> as shown in [Table 8] below, and PCR was performed under the conditions shown in [Table 9] below. Next, purification of the PCR-completed sample was performed using HiAccuBead according to the manufacturer's procedures.

**[Table 8]**

| Reagent | Amount for one time |
|---|---|
| Microbial Genomic DNA (12 ng/µL) | 2.5 µL |
| Primer 1 in [Table 4] (0.5 µM) | 5 µL |
| Mixture of primers 2 to 9 in [Table 4] (0.5 µM each) | 5 µL |
| 2X KAPA HiFi Hotstart ReadyMix (Roche) | 12.5 µL |
| Total amount | 25 µL |

**[Table 9]**

| Step | Temperature | Time | Number of cycles |
|---|---|---|---|
| Initial denaturation | 95°C | 3 minutes | 1 cycle |
| Denaturation | 95°C | 30 seconds | 25 cycles |
| Annealing | 55°C | 30 seconds | |
| Extension | 72°C | 30 seconds | |
| Elongation | 72°C | 5 minutes | 1 cycle |
| Preservation | 4°C | - | - |

Index PCR was performed using the sample obtained by the Amplicon PCR as follows. A PCR reaction solution was prepared using the Nextera XT Index Kit v2 (Illumina, #20528300) according to the manufacturer's procedures as shown in [Table 10] below, and PCR was performed under the conditions shown in [Table 11] below. Next, purification of the PCR-completed sample was performed using HiAccuBead according to the manufacturer's procedures.

**[Table 10]**

| Reagent | Amount for one time |
|---|---|
| 10 mM Tris pH 8.5 | 10 µL |
| 2X KAPA HiFi Hotstart ReadyMix | 25 µL |
| Nextera XT Index 1 primer | 5 µL |
| Nextera XT Index 2 primer | 5 µL |
| Sample | 5 µL |
| Total amount | 50 µL |

**[Table 11]**

| Step | Temperature | Time | Number of cycles |
|---|---|---|---|
| Initial denaturation | 95°C | 3 minutes | 1 cycle |
| Denaturation | 95°C | 30 seconds | 8 cycles |
| Annealing | 55°C | 30 seconds | |
| Extension | 72°C | 30 seconds | |
| Elongation | 72°C | 5 minutes | 1 cycle |
| Preservation | 4°C | - | - |

Sequencing of the DNA library obtained by the index PCR was performed as follows. The DNA library obtained by the index PCR was diluted to have a concentration of 100 nM and then mixed with 100 nM phiX Control v3 (Illumina, RGT25975053) at a ratio of 7:3. The DNA library was loaded according to the system guide of the iSeq100 equipment, and sequencing was performed with 300 bp single read to acquire a fastq file. Next, OTU (Operational Taxonomic Unit) distribution analysis was performed using the acquired fastq file according to the procedures of the CLC Microbial Genomics Module of CLC Genomics Workbench (Qiagen) to confirm 19 probiotic species notified by the Ministry of Food and Drug Safety, 2 probiotic species added in food, and 4 pathogenic bacterial species in [Table 1]. Specifically, the detection ability depending on the number of viable bacteria was examined as shown in [Table 12] below through two repeated tests, and the detection ability depending on the ratio of each species contained in the test specimen was examined as shown in [Table 13] below.

**[Table 12]**

| No. | Species name | Decision result by number of viable bacteria | | | | | |
|---|---|---|---|---|---|---|---|
| | | 10³ | 10⁴ | 10⁵ | 10⁶ | 10⁷ | 10⁸ |
| 1 | *Lactobacillus acidophilus* | + | + | + | + | + | + |
| 2 | *Lactobacillus gasseri* | - | + | + | + | + | + |
| 3 | *Lactobacillus helveticus* | - | + | + | + | + | + |
| 4 | *Lacticaseibacillus paracasei*/*casei*/*rhamnosus* | + | + | + | + | + | + |
| 5 | *Limosilactobacillus fermentum* | - | - | + | + | + | + |
| 6 | *Limosilactobacillus reuteri* | - | + | + | + | + | + |
| 7 | *Lactiplantibacillus plantarum* | - | + | + | + | + | + |
| 8 | *Ligilactobacillus salivarius* | - | + | + | + | + | + |
| 9 | *Lactococcus lactis* | - | + | + | + | + | + |
| 10 | *Enterococcus faecium* | - | + | + | + | + | + |
| 11 | *Enterococcus faecalis* | - | + | + | + | + | + |
| 12 | *Streptococcus thermophilus* | + | + | + | + | + | + |
| 13 | *Bifidobacterium bifidum* | - | - | + | + | + | + |
| 14 | *Bifidobacterium breve* | - | + | + | + | + | + |
| 15 | *Bifidobacterium animalis ssp. Lactis* | - | - | + | + | + | + |
| 16 | *Lactobacillus delbruecki ssp. Bulgaricus* | - | + | + | + | + | + |
| 17 | *Bifidobacterium longum* | - | + | + | + | + | + |
| 18 | *Latilactobacillus curvatus* | + | + | + | + | + | + |
| 19 | *Bacillus coagulans* | + | + | + | + | + | + |
| 20 | *Escherichia coli* | - | + | + | + | + | + |
| 21 | *Salmonella spp.* | + | + | + | + | + | + |
| 22 | *Staphylococcus aureus* | - | + | + | + | + | + |
| 23 | *Pseudomonas aeruginosa* | + | + | + | + | + | + |

**[Table 13]**

| No. | Species name | Decision result by ratio of bacteria contained in test specimen | | | | |
|---|---|---|---|---|---|---|
| | | 0.01 | 0.1 | 1 | 5 | 10 |
| 1 | *Lactobacillus acidophilus* | - | *+* | *+* | *+* | *+* |
| 2 | *Lactobacillus gasseri* | - | *+* | *+* | *+* | *+* |
| 3 | *Lactobacillus helveticus* | - | *+* | *+* | *+* | *+* |
| 4 | *Lacticaseibacillus paracasei*/*casei*/*rhamnosus* | *+* | *+* | *+* | *+* | *+* |
| 5 | *Limosilactobacillus fermentum* | - | *+* | *+* | *+* | *+* |
| 6 | *Limosilactobacillus reuteri* | - | *+* | *+* | *+* | *+* |
| 7 | *Lactiplantibacillus plantarum* | - | *+* | *+* | *+* | *+* |
| 8 | *Ligilactobacillus salivarius* | - | *+* | *+* | *+* | *+* |
| 9 | *Lactococcus lactis* | - | *+* | *+* | *+* | *+* |
| 10 | *Enterococcus faecium* | - | *+* | *+* | *+* | *+* |
| 11 | *Enterococcus faecalis* | - | *+* | *+* | *+* | *+* |
| 12 | *Streptococcus thermophilus* | - | *+* | *+* | *+* | *+* |
| 13 | *Bifidobacterium bifidum* | - | *+* | *+* | *+* | *+* |
| 14 | *Bifidobacterium breve* | - | *+* | *+* | *+* | *+* |
| 15 | *Bifidobacterium animalis ssp. Lactis* | - | *+* | *+* | *+* | *+* |
| 16 | *Lactobacillus delbruecki ssp. Bulgaricus* | - | *+* | *+* | *+* | *+* |
| 17 | *Bifidobacterium longum* | - | *+* | *+* | *+* | *+* |
| 18 | *Latilactobacillus curvatus* | *+* | *+* | *+* | *+* | *+* |
| 19 | *Bacillus coagulans* | *+* | *+* | *+* | *+* | *+* |
| 20 | *Escherichia coli* | *+* | *+* | *+* | *+* | *+* |
| 21 | *Salmonella spp.* | *+* | *+* | *+* | *+* | *+* |
| 22 | *Staphylococcus aureus* | - | *+* | *+* | *+* | *+* |
| 23 | *Pseudomonas aeruginosa* | *+* | *+* | *+* | *+* | *+* |

In the decision results by the bacterial species identification method, - means that the species was introduced into the test specimen but the bacteria were not detected by the species discrimination method, and + means that the bacteria were successfully detected.

As a result, as shown in Tables 12 and 13, it has been confirmed that the detection limit for discriminating all the 19 probiotic species notified by the Ministry of Food and Drug Safety, 2 probiotic species added in food, and 4 pathogenic bacterial species in a mixed strain suspension is 10⁵ CFU/mL or more in terms of the number of viable bacteria and 0.1% in terms of the ratio of bacteria contained in the test specimen.

### <Example 5> Discrimination of bacterial species in mixed strain suspension containing auxiliary material

It was examined whether bacterial species in a mixed strain suspension containing an auxiliary material could be discriminated using the fusion primer sets of <Example 1> and the bacterial species discrimination method of <Example 3>.

Specifically, the 19 probiotic species notified by the Ministry of Food and Drug Safety, 2 probiotic species added in food, and 4 pathogenic bacterial species, which were listed in [Table 7], were cultured according to the methods suggested by the distributors, then the number of viable bacteria was measured by the dispensing well plate method, and the bacteria were diluted with PBS to have a concentration of 5 × 10⁶ CFU/mL and mixed together to prepare a mixture of 25 bacterial species, which were mixed at the same CFU. To the prepared mixture, 2 g of indigestible maltodextrin was added to prepare a mixed strain suspension containing an auxiliary material. Next, the 25 bacterial species were confirmed in the same manner as in <Example 3>.

As a result, all the 19 probiotic species notified by the Ministry of Food and Drug Safety, 2 probiotic species added in food, and 4 pathogenic bacterial species, which are mixed in a test specimen, have been successfully discriminated regardless of whether additives are contained, and it has been confirmed that this method can be applied to test specimens containing auxiliary materials.

**<Example 6> Discrimination of bacterial species in probiotic product using primer set for discrimination of bacterial species**

It was examined whether that 19 probiotic species notified by the Ministry of Food and Drug Safety, 2 probiotic species added in food, and 4 pathogenic bacterial species in probiotic-containing products sold domestically and overseas could be discriminated using the fusion primer sets of <Example 1> and the bacterial species discrimination method of <Example 3>. [Table 14] shows the results acquired by repeatedly performing bacterial species discrimination on five types of probiotic products two times.

**[Table 14]**

| No. | Species name | Product name | | | | |
|---|---|---|---|---|---|---|
| | | A | B | C | D | E |
| 1 | *Lactobacillus acidophilus* | *+* | *-* | *-* | *+* | *-* |
| 2 | *Lactobacillus gasseri* | *-* | *+* | *-* | *-* | *-* |
| 3 | *Lactobacillus helveticus* | *-* | *-* | *-* | *+* | *-* |
| 4 | *Lacticaseibacillus paracasei*/*casei*/*rhamnosus* | *-* | *-* | *-* | *+* | *-* |
| 5 | *Limosilactobacillus fermentum* | *-* | *-* | *-* | *-* | *-* |
| 6 | *Limosilactobacillus reuteri* | *-* | *-* | *-* | *-* | *-* |
| 7 | *Lactiplantibacillus plantarum* | *+* | *-* | *+* | *+* | *+* |
| 8 | *Ligilactobacillus salivarius* | *-* | *-* | *-* | *-* | *-* |
| 9 | *Lactococcus lactis* | *-* | *-* | *-* | *-* | *+* |
| 10 | *Enterococcus faecium* | *+* | *-* | *-* | *-* | *-* |
| 11 | *Enterococcus faecalis* | *-* | *-* | *-* | *-* | *-* |
| 12 | *Streptococcus thermophilus* | *+* | *-* | *-* | *+* | *+* |
| 13 | *Bifidobacterium bifidum* | *-* | *-* | *-* | *-* | *-* |
| 14 | *Bifidobacterium breve* | *-* | *-* | *-* | *+* | *+* |
| 15 | *Bifidobacterium animalis ssp. Lactis* | *+* | *-* | *-* | *+* | *-* |
| 16 | *Lactobacillus delbruecki ssp. Bulgaricus* | *-* | *-* | *-* | *-* | *-* |
| 17 | *Bifidobacterium longum* | *-* | *-* | *-* | *-* | *+* |
| 18 | *Latilactobacillus curvatus* | *-* | *-* | *+* | *-* | *-* |
| 19 | *Bacillus coagulans* | *-* | *-* | *-* | *-* | *-* |
| 20 | *Escherichia coli* | *-* | *-* | *-* | *-* | *-* |
| 21 | *Salmonella spp.* | *-* | *-* | *-* | *-* | *-* |
| 22 | *Staphylococcus aureus* | *-* | *-* | *-* | *-* | *-* |
| 23 | *Pseudomonas aeruginosa* | *-* | *-* | *-* | *-* | *-* |

In the decision results by the bacterial species identification method, - means that the ingredient is not listed on the product and has not been detected as a result of bacterial species discrimination, and + means that the ingredient is listed on the product and has been detected as a result of bacterial species discrimination.

As a result, as shown in Table 14, it has been confirmed that the detected probiotic species match the probiotic ingredients listed on the products.

Through the results, it has been confirmed that all probiotic ingredients listed on the products can be discriminated using the primer sets and the method for discriminating bacterial species using the same of the present invention and that the discrimination of bacterial species in probiotic-containing products sold domestically and overseas can be performed quickly and at low cost.

### [Industrial Applicability]

It has been confirmed that 21 probiotic species and 4 pathogenic bacterial species can be discriminated with high resolution and accuracy using the primer set for discrimination of bacterial species in a probiotic composition according to the present invention, so the primer set can be usefully used to discriminate bacterial species in a probiotic composition.

## Claims

1. A primer set for discrimination of bacterial species in a probiotic composition, the primer set comprising primers represented by SEQ ID NOS: 1 to 9.

2. The primer set for discrimination of bacterial species in a probiotic composition according to claim 1, wherein the primers further contain an adapter sequence at a 5' end.

3. The primer set for discrimination of bacterial species in a probiotic composition according to claim 2, wherein the primers are represented by SEQ ID NOS: 10 to 18.

4. The primer set for discrimination of bacterial species in a probiotic composition according to claim 1, wherein the primer set targets an ITS (internal transcribed spacer) region of a bacterial species.

5. The primer set for discrimination of bacterial species in a probiotic composition according to claim 1, wherein the bacterial species are *Lactobacillus acidophilus, Lactobacillus gasseri, Lactobacillus delbruecki* ssp. *Bulgaricus, Lactobacillus helveticus, Lacticaseibacillus casei, Lacticaseibacillus paracasei, Lacticaseibacillus rhamnosus, Limosilactobacillus fermentum, Limosilactobacillus reuteri, Lactiplantibacillus plantarum, Ligilactobacillus salivarius, Lactococcus lactis, Enterococcus faecium, Enterococcus faecalis, Streptococcus thermophilus, Bifidobacterium bifidum, Bifidobacterium breve, Bifidobacterium longum, Bifidobacterium animalis* ssp. *lactis, Latilactobacillus curvatus, Bacillus coagulans, Escherichia coli, Salmonella* spp., *Staphylococcus aureus* and *Pseudomonas aeruginosa.*

6. A composition for discrimination of bacterial species in a probiotic composition, the composition comprising the primer set according to any one of claims 1 to 5.

7. A method for discriminating bacterial species in a probiotic composition, the method comprising:
1) isolating DNA from the probiotic composition;
2) performing PCR using the DNA isolated in step 1) as a template and the primer set according to any one of claims 1 to 5 to amplify a target sequence; and
3) detecting the amplification product obtained in step 2).

8. The method for discriminating bacterial species in a probiotic composition according to claim 7, further comprising performing index PCR using the amplification product obtained in step 2) as a template.

9. The method for discriminating bacterial species in a probiotic composition according to claim 7, wherein detection of the PCR amplification product is performed through sequencing.
